(19) Europäisches Patentamt
European Patent Office
Office européen des brevets

(11) Numéro de publication : **0 335 777 B1**

(12) # FASCICULE DE BREVET EUROPEEN

(45) Date de publication du fascicule du brevet : **18.12.91 Bulletin 91/51**

(51) Int. Cl.$^5$ : **A61K 7/42**, A61K 7/48, A61K 7/06

(21) Numéro de dépôt : **89400815.0**

(22) Date de dépôt : **23.03.89**

(54) **Utilisation cosmétique de diorganopolysiloxanes à fonction benzylidène-3 camphre.**

Le dossier contient des informations techniques présentées postérieurement au dépôt de la demande et ne figurant pas dans le présent fascicule.

(30) Priorité : **28.03.88 LU 87180**

(43) Date de publication de la demande : **04.10.89 Bulletin 89/40**

(45) Mention de la délivrance du brevet : **18.12.91 Bulletin 91/51**

(84) Etats contractants désignés : **AT BE CH DE ES FR GB GR IT LI NL SE**

(56) Documents cités :
**EP-A- 0 138 590**
**GB-A- 2 188 320**
**SEIFEN-ÖLE-FETTE-WACHSE, vol. 114, no. 2, 4 février 1988, pages 51-54, Augsburg, DE; J. ROIDL: "Bedeutung der Silicone in der modernen Kosmetik"**

(73) Titulaire : **L'OREAL**
**14, Rue Royale**
**F-75008 Paris (FR)**

(72) Inventeur : **Grollier, Jean-François**
**16, bis Boulevard Morland**
**F-75004 Paris (FR)**
Inventeur : **Richard, Hervé**
**48, Rue de L'Ermitage**
**F-75020 Paris (FR)**
Inventeur : **Forestier, Serge**
**16, Allée Ferdinand Buisson**
**F-77410 Claye-Souilly (FR)**
Inventeur : **Lang, Gérard**
**44 Avenue Lacour**
**F-95210 Saint-Gratien (FR)**

(74) Mandataire : **Casalonga, Axel et al**
**BUREAU D.A. CASALONGA - JOSSE**
**Morassistrasse 8**
**W-8000 München 5 (DE)**

EP 0 335 777 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention est relative à l'utilisation en cosmétique, notamment en tant qu'agents filtrant le rayonnement UV, de diorganopolysiloxanes à fonction benzylidène-3 camphre ainsi qu'aux nouvelles compositions cosmétiques contenant ces composés, destinées à la protection de la peau et des cheveux.

On sait que les radiations lumineuses de longueurs d'onde comprises entre 280 nm et 400 nm permettent le brunissement de l'épiderme humain et que les rayons de longueurs d'onde comprises entre 280 et 320 nm, connues sous la dénomination d'UV-B, provoquent des érythèmes et des brûlures cutanées qui peuvent nuire au développement du bronzage ; ce rayonnement UV-B doit donc être filtré.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques.

Il est donc intéressant de disposer de composés absorbant les rayons UV sur une large bande afin de pouvoir filtrer à la fois les rayons UV-A et UV-B.

On sait par ailleurs que les constituants entrant dans les préparations cosmétiques ne possèdent pas toujours une stabilité suffisante à la lumière et qu'ils se dégradent sous l'action des radiations lumineuses.

Par conséquent, il est souhaitable d'incorporer à ces préparations des composés susceptibles de filtrer les rayons UV et qui doivent présenter en outre une bonne stabilité et une solubilité suffisante dans les milieux habituellement utilisés en cosmétique, et en particulier dans les huiles et graisses.

Il est également souhaitable d'assurer aux cheveux une bonne protection contre la dégradation photochimique afin déviter en particulier une décoloration ou un changement de nuance.

On sait, par ailleurs, greffer sur des chaînes de polymères carbonés synthétiques, de polymères naturels, d'hydrolysats de protéines ou de polyaminoamides, des restes de molicules ayant un effet filtre vis-à-vis du rayonnement UV ; ces polymères greffés décrits par exemple dans les brevets français n° 2197023, 2237912, 2531960, 2548018, 2549069, 2586692 et 2586693 peuvent être utilisés pour préparer des compositions cosmétiques protectrices de l'épiderme humain ou anti-solaires. On a cependant constaté que ces polymères greffés sont généralement peu solubles dans les solvants cosmétiques usuels, notamment dans les supports gras, et qu'ils forment des films dont la structure est trop rigide.

On connait également des composés silanes ou siloxanes greffés par des fonctions méthallylcinnamate, méthallylsalicylate ou méthyallyl-N,N-dialkylaminobenzoate décrits dans la demande de brevet européen EP-A-0138590. Ces composés ont des propriétés filtrantes dans le domaine des rayons UV-B.

Des polymères poly(acrylamidométhyle) greffés par une fonction alcoxybenzylidène-3 camphre ayant des propriétés filtrantes dans une large gamme de longueurs d'onde allant de 280 à 370 nm ont été décrits dans la demande de brevet GB 2188320.

La demanderesse a découvert que certains diorganopolysiloxanes à fonction benzylidène-3 camphre présentaient, de manière étonnante, de bonnes propriétés cosmétiques associées à de bonnes propriétés filtrantes dans une large gamme de longueurs d'onde allant de 280 à 360 nm. Ils présentent notamment un excellent caractère liposoluble, ce qui les rend utilisables dans les supports gras utilisés en cosmétique. Outre leur bon pouvoir filtrant et leur bonne solubilité dans les corps gras et les solvants cosmétiques usuels, ces diorganopolysiloxanes à fonction benzylidène-3 camphre présentent une excellente stabilité chimique et photochimique et ont l'avantage d'apporter de la douceur à la peau et aux cheveux, par lesquels ils sont bien tolérés.

La présente invention a donc pour objet l'utilisation en cosmétique, en particulier en tant qu'agents filtrant le rayonnement UV de longueurs d'onde comprises entre 280 et 360 nm, de diorganopolysiloxanes à fonction benzylidène-3 camphre choisis parmi ceux de formule :

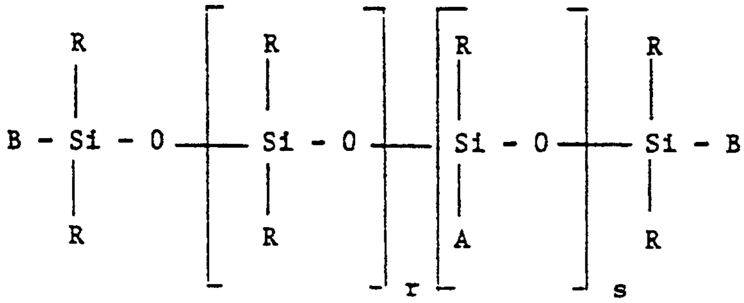

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre entier choisi entre 0 et 200 inclusivement,

s est un nombre entier choisi entre 0 et 50 inclusivement et si s est 0, au moins l'un des deux symboles B désigne A, et ceux de formule :

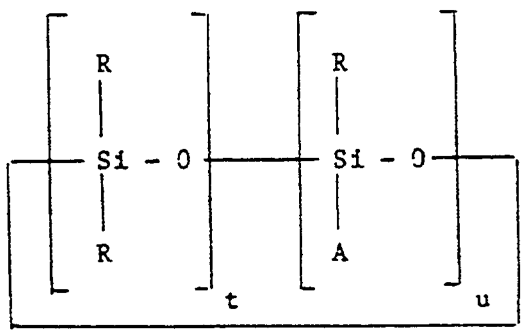

(2)

dans laquelle

R a la même signification qu'à la formule (1),

u est un nombre entier compris entre 1 et 20 inclus et

t est un nombre entier compris entre 0 et 20 inclus,

t + u est égal ou supérieur à 3, formules dans lesquelles le symbole A est un radical de formule :

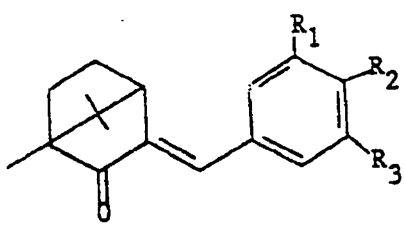

(3)

dans laquelle :

$R_1$ et $R_2$ représentent un atome d'hydrogène, un radical alkyle en $C_1$-$C_6$, un radical alcoxy en $C_1$-$C_6$, hydroxyle, triméthylsilyloxy ou un radical divalent Y de formule :

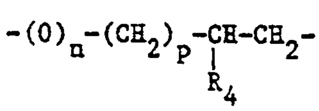

,

l'un des deux radicaux $R_1$ et $R_2$ représentant nécessairement le radical divalent Y,

$R_4$ représente un atome d'hydrogène ou un radical alkyle en $C_1$-$C_4$,

n est 0 ou 1,

p est un nombre entier compris entre 1 et 10 inclus,

$R_3$ représente un atome d'hydrogène, un radical alkyle en $C_1$-$C_6$ ou un alcoxy en $C_1$-$C_6$.

On utilise plus particulièrement les polymères statistiques ou à blocs de formules (1) ou (2) présentant au moins l'une des caractéristiques suivantes :

— R est méthyle,

— B est méthyle,

— r est compris entre 5 et 20 inclus,

— s est compris entre 2 et 15 inclus,

— t + u est compris entre 3 et 10 inclus,

— $R_1$ est H ou méthoxy,

— $R_2$ est H ou méthoxy,

l'un des deux radicaux $R_1$ ou $R_2$ représentant le radical divalent Y dans lequel n = 0 ou 1, p = 1, $R_4$ = H ou méthyle,

— $R_3$ est H ou méthoxy.

Les polymères de formule (1) et (2) peuvent être préparés à partir de polymères dans lesquels tous les radicaux A sont des atomes d'hydrogène, dénommés polymères à SiH, de formules (4) et (5) :

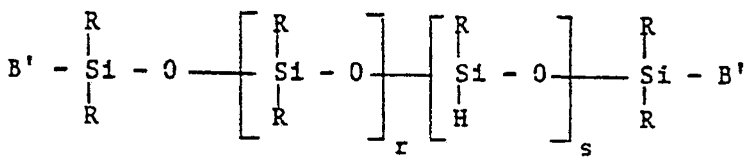

(4)

où R, r et s ont la signification donnée pour la formule (1) et les radicaux B', identiques ou différents, sont choisis parmi les radicaux R et un atome d'hydrogène,

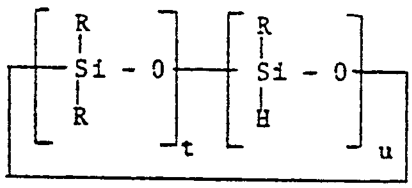

(5)

où R, t et u ont la signification donnée dans la formule (2).

A l'aide de ces polymères à SiH de formules (4) ou (5), on effectue une réaction d'hydrosilylation en présence d'une quantité catalytiquement efficace d'un catalyseur au platine sur un dérivé organique de benzylidène-3 camphre de formule :

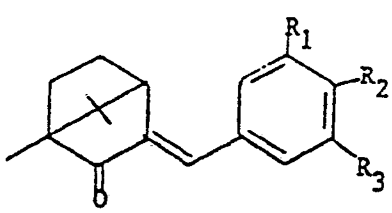

(6)

dans laquelle $R_1$, $R_2$ et $R_3$ ont la même signification qu'à la formule (3) sauf que le radical Y est alors le radical homologue insaturé Y' monovalent de formule :

$$—(O)_n-(CH_2)_p-C(R_4) = CH_2$$

dans laquelle n, p et $R_4$ ont la même signification qu'à la formule (3).

Les composés de formule (6) dans laquelle $R_2$ représente le groupe $—O(CH_2)_p-C(R_4) = CH_2$ peuvent être préparés comme indiqué dans le brevet français n° 2430938, par réaction de l'halogénure d'alcényle correspondant sur le p-hydroxybenzylidène-3 camphre correspondant, en présence de carbonate de sodium dans le diméthylformamide. Les composés de formule (6) dans laquelle $R_1$ représente le groupe $—(O)_n-(CH_2)_p-C(R_4) = CH_2$ ou $R_2$ le groupe $—(CH_2)_p-C(R_4) = CH_2$ peuvent être préparés selon l'un des procédés suivants :

1er procédé :

Préparation des composés de formule (6) dans laquelle $R_1$ représente un radical $-(O)_n-(CH_2)_p-C(R_4)=CH_2$ ou bien $R_2$ représente un radical $-(CH_2)_p-C(R_4)=CH_2$, où $R_1$, $R_2$, n, p, $R_4$ et $R_3$ ont les significations indiquées ci-dessus.

On condense un aldéhyde aromatique de formule (II) sur le camphre synthétique (d, 1-camphre) ou le camphre naturel (d-camphre) selon le schéma réactionnel suivant :

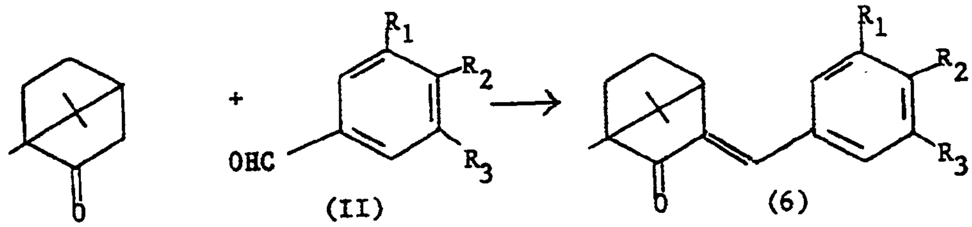

(II) (6)

Cette réaction est effectuée en présence d'une base, par exemple en présence d'un amidure, d'un hydrure ou d'un alcoolate de métal alcalin, dans un solvant inerte tel que le benzène, le toluène, l'éther, le dioxane ou le diméthoxy-1,2-éthane, à une température comprise entre 0°C et le point d'ébullition du solvant.

a) L'aldéhyde de formule (II) dans laquelle $R_1$ représente un radical $-(O)_n-(CH_2)_p-C(R_4)=CH_2$ lorsque n = 1, peut être obtenu par réaction d'un halogénure d'alcényle de formule (III) sur un aldéhyde de formule (IIA) selon le schéma réactionnel suivant :

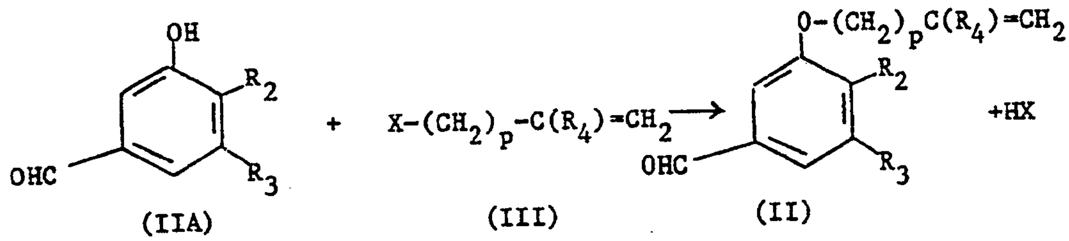


(IIA) (III) (II)

Cette réaction est effectuée en présence d'une base dans un solvant, par exemple en présence d'un carbonate ou hydroxyde de métal alcalin ou alcalino-terreux ou d'un amidure, hydrure ou alcoolate alcalin, dans un solvant compatible avec la nature de la base tel que l'eau ou un solvant organique comme le diméthylformamide, le diméthylsulfoxyde, le dioxane ou un alcool, à une température comprise entre la température ambiante et le point d'ébullition du solvant.

Dans l'aldéhyde de formule (IIA), qui peut être préparé selon des méthodes connues, et dans l'aldéhyde de formule (II) dans laquelle $R_1$ représente un radical $-O-(CH_2)_p-C(R_4)=CH_2$, $R_2$ et $R_3$ représentent un atome d'hydrogène, un radical alkyle en $C_1$-$C_6$ ou un radical alcoxy en $C_1$-$C_6$. Dans le composé de formule (III), X représente un atome d'halogène, de préférence le chlore ou le brome, et $R_4$ et p ont les significations mentionnées ci-dessus.

b) L'aldéhyde de formule (II) dans laquelle $R_1$ représente un radical $-(O)_n-(CH_2)_p-C(R_4)=CH_2$ lorsque n = 0, ou dans laquelle $R_2$ représente un radical $-(CH_2)_p-C(R_4)=CH_2$, peut être obtenu par réaction de l'orthoformiate d'éthyle sur un bromure de phénylmagnésium de formule (IV), suivie d'une hydrolyse de l'acétal formé, selon le schéma réactionnel suivant :

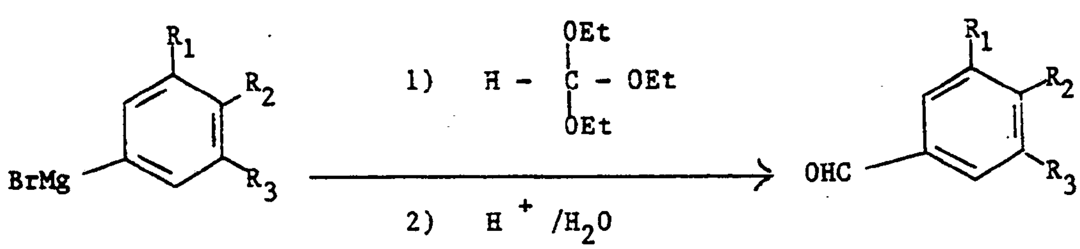


(IV) (IIB)

Cette réaction peut être effectuée dans les conditions décrites par QUELET (C.R. Acad. Science Vol. 182, p. 1285 et Bull. Soc. Chim. Fr. vol. 45, p. 267), par exemple dans un solvant inerte tel que l'éther éthylique, le dioxane ou le diméthoxy-1,2-éthane, à une température comprise entre la température ambiante et le point d'ébullition du solvant.

Dans les composés de formule (IIB) et (IV), l'un des substituants $R_1$ ou $R_2$ représente un radical $(CH_2)_p$-$C(R_4)=CH_2$, $R_4$ et p ayant la signification mentionnée ci-dessus, et l'autre représente un atome d'hydrogène, un radical alkyle en $C_1$-$C_6$ ou un radical alcoxy en $C_1$-$C_6$ et $R_3$ représente un atome d'hydrogène, un radical alkyle en $C_1$-$C_6$ ou un radical alcoxy en $C_1$-$C_6$.

2ème Procédé

Préparation des composés de formule (6) dans laquelle $R_1$ représente un radical $-(O)_n-(CH_2)_p-C(R_4)=CH_2$ lorsque n est égal à 1 (composés IA)

Ces composés peuvent être obtenus par réaction d'un halogénure d'alcényle de formule (III) sur un hydroxy-3'-benzylidène-3 camphre de formule (V), en présence d'une base par exemple en présence d'un hydroxyde ou carbonate de métal alcalin ou alcalino-terreux ou d'un amidure, alcoolate ou hydrure alcalin, dans un solvant compatible avec la nature de la base tel que l'eau ou un solvant organique tel qu'un alcool, le dioxane, le diméthylsulfoxyde ou le diméthylformamide, à une température comprise entre la température ambiante et le point d'ébullition du solvant, selon le schéma réactionnel suivant :

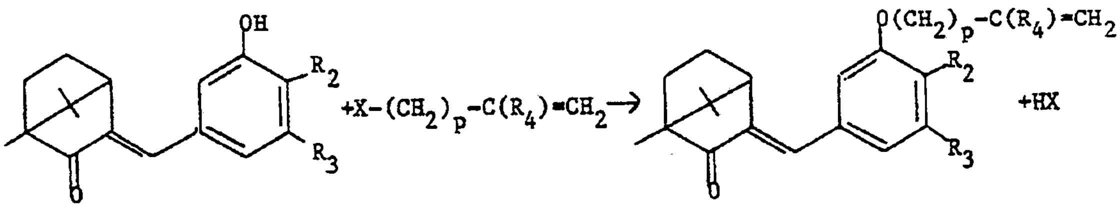

(V) (III) (IA)

Dans les composés de formules (V), (III) et (IA), $R_2$ et $R_3$ représentent un atome d'hydrogène, un radical alkyle en $C_1$-$C_6$ ou un radical alcoxy en $C_1$-$C_6$, $R_4$ et p ont la signification mentionnée ci-dessus et X représente un atome d'halogène, de préférence le chlore ou le brome.

Les composés de formule (V) peuvent être obtenus par hydrolyse acide des composés de formule (VI) dans laquelle $R_5$ représente un reste alkyle en $C_1$-$C_6$, de préférence un reste méthyle, et $R_2$ et $R_3$ désignent un atome d'hydrogène ou un radical alkyle ou alcoxy en $C_1$-$C_6$, selon le schéma réactionnel suivant :

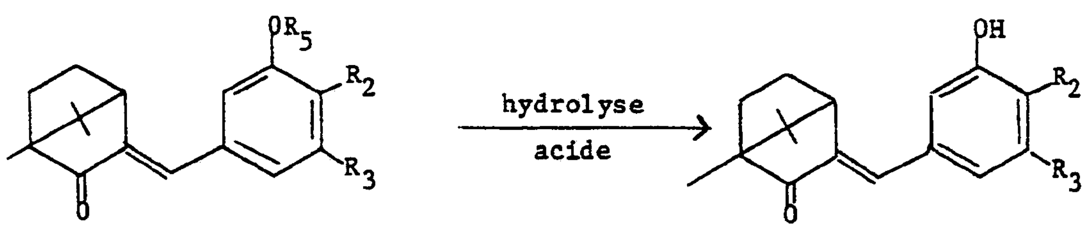


(VI) (V)

Cette hydrolyse peut être réalisée en présence d'un agent d'hydrolyse comme par exemple le chlorhydrate de pyridine, à une température voisine du point d'ébullition du mélange réactionnel.

Les composés de formule (V) peuvent également être préparés par condensation d'un aldéhyde de formule (IIA) sur le camphre synthétique (d, 1-camphre) ou naturel (d-camphre), selon le schéma réactionnel suivant:

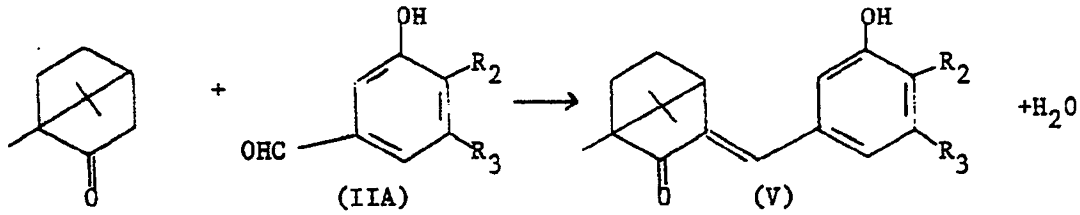

(IIA) (V)

$R_2$ et $R_3$ désignant un atome d'hydrogène, un radical alkyle ou alcoxy en $C_1$-$C_6$.

Cette réaction est effectuée en présence d'une base dans un solvant, par exemple au moyen d'hydrure de sodium ou de tertiobutylate de potassium dans le dioxane ou le diméthoxy-1,2-éthane, à une température comprise entre la température ambiante et le point d'ébullition du solvant.

3ème procédé

Préparation des composés de formule (6) dans laquelle $R_1$ représente un radical $-(O)_n-(CH_2)_p-C(R_4)=CH_2$ lorsque n = 0 et p = 1 (composés ID).

Ces composés peuvent être obtenus par réarrangement de CLAISEN d'un composé de formule (IB) selon le schéma réactionnel ci-dessous :

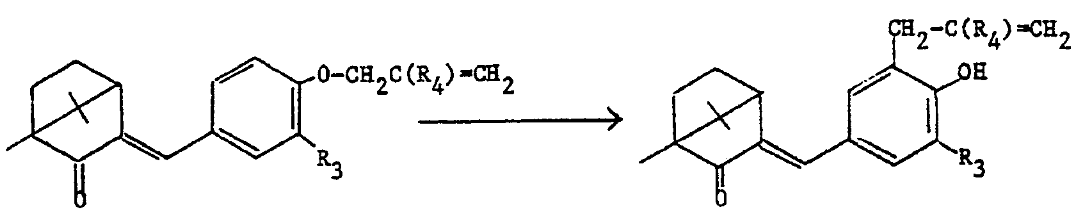

(IB) (IC)

Dans les composés (IB) et (IC), $R_3$ et $R_4$ ont les significations mentionnées ci-dessus.

Le composé (IB) peut être préparé de façon connue, en faisant réagir un hydroxy-4'-benzylidène-3 camphre, de formule (VII) sur un halogénure d'alcényle de formule (III) dans laquelle p=1 selon le schéma :

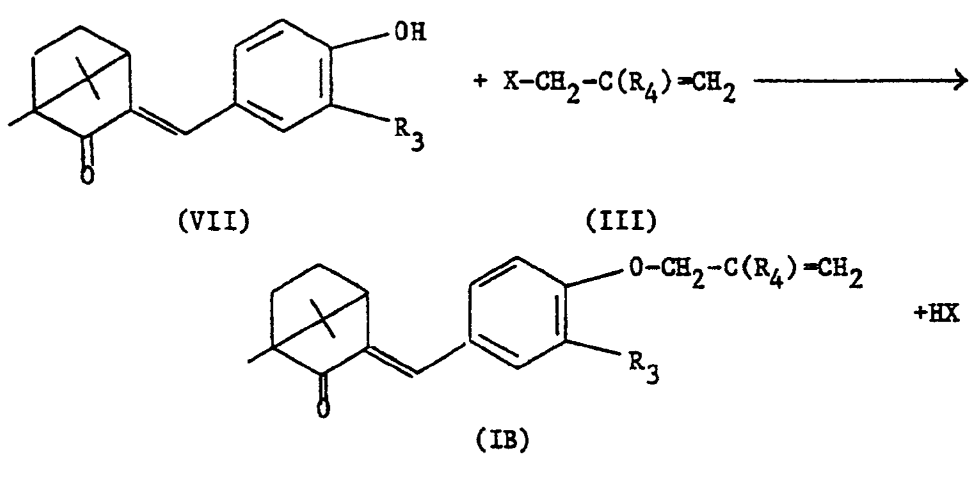

(VII) (III)

(IB)

$R_3$ et $R_4$ ayant les significations mentionnées ci-dessus et X étant un halogène de préférence le chlore ou le brome. Cette réaction a lieu en présence d'une base, par exemple un carbonate ou hydroxyde de métal alcalin ou alcalino-terreux, un amidure, alcoolate ou hydrure alcalin, dans un solvant compatible avec la nature de la base tel que l'eau ou un solvant organique, par exemple un alcool, le dioxane, le diméthylsulfoxyde, ou le diméthylformamide, à une température comprise entre la température ambiante et le point d'ébullition du solvant.

Le réarrangement de CLAISEN peut être effectué dans les conditions décrites par TARBELL (Organic Reactions, Vol. 2, John Wiley, New York, 1944, page 1), par chauffage à au moins 170°C environ du composé de formule (IB), éventuellement en présence d'un solvant.

Le composé de formule (IC) ainsi obtenu est transformé en composé de formule (ID) dans laquelle $R_2$ représente un radical alcoxy en $C_1$-$C_6$ par réaction avec un halogénure d'alkyle en $C_1$-$C_6$ en présence d'une base, par exemple un carbonate de métal alcalin, dans un solvant tel que le diméthylformamide, ou bien en présence d'un hydrure de métal alcalin dans le diméthoxy-1,2-éthane suivant le schéma réactionnel suivant :

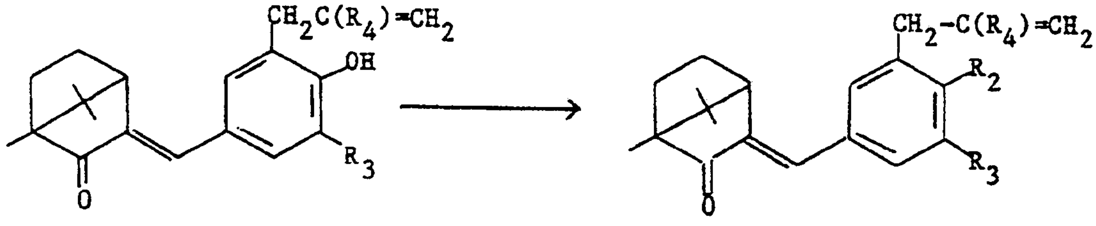

(IC) (ID)

Dans le composé (ID), $R_2$ représente un radical alcoxy en $C_1$-$C_6$ et $R_3$ et $R_4$ ont la signification mentionnée ci-dessus.

Les catalyseurs au platine utilisés pour réaliser la réaction d'hydrosilylation des polymères de formules (4) ou (5) sur le dérivé organique de formule (6) sont amplement décrits dans la littérature. On peut en particulier citer les complexes du platine et d'un produit organique décrit dans les brevets américains US-A-3159601, US-A-3159602, US-A-3220972 et les brevets européens EP-A-57459, EP-A-188978 et EP-A-190530 et les complexes du platine et d'organopolysiloxane vinylé décrits dans les brevets américains US-A-3419593, US-A-3715334, US-A3377432 et US-A-3814730.

Pour faire réagir le polymère à SiH de formule (4) ou (5) sur le dérivé de formule (6), on utilise généralement une quantité de catalyseur au platine, calculée en poids de platine métal, comprise entre 5 et 600 ppm, de préférence entre 10 et 200 ppm, basée sur le poids de polymère à SiH de formule (4) ou (5).

La réaction d'hydrosilylation peut avoir lieu en masse ou au sein d'un solvant organique volatil tel que le toluène, l'heptane, le xylène, le tétrahydrofuranne et le tétrachloréthylène.

Il est généralement souhaitable de chauffer le mélange réactionnel à une température de 60 à 120°C pendant le temps nécessaire pour que la réaction soit complète. Par ailleurs, il est souhaitable d'ajouter goutte à goutte le polymère à SiH sur le dérivé de formule (6) en solution dans un solvant organique.

On vérifie que la réaction est complète en dosant les SiH résiduels par la potasse alcoolique, puis on élimine le solvant, par exemple par distillation sous pression réduite.

L'huile brute obtenue peut être purifiée, par exemple par passage sur une colonne absorbante de silice.

Un autre objet de l'invention est constitué par les compositions cosmétiques destinées à protéger la peau et les cheveux du rayonnement UV, contenant une quantité efficace d'un diorganopolysiloxane à fonction benzylidène-3-camphre de formule (1) ou (2), dans un milieu cosmétiquement acceptable.

La présente invention a également pour objet un procédé de protection de la peau et des cheveux naturels ou sensibilisés vis-à-vis du rayonnement solaire, consistant à appliquer sur la peau ou les cheveux une quantité efficace d'au moins un composé de formule (1) ou (2) contenu dans un support cosmétiquement acceptable comprenant au moins une phase grasse.

On entend par "cheveux sensibilisés" des cheveux ayant subi un traitement de permanente, de coloration ou de décoloration.

L'invention a également pour objet une composition cosmétique colorée ou non colorée, stabilisée à la lumière, comprenant une quantité efficace d'au moins un diorganopolysiloxane à fonction benzylidène-3 camphre de formule (1) ou (2) ci-dessus.

Lorsqu'elle est utilisée comme composition destinée à protéger l'épiderme humain contre les rayons ultraviolets, la composition cosmétique selon l'invention peut se présenter sous les formes les plus diverses habituellement utilisées pour ce type de composition. Elle peut notamment se présenter sous forme de lotions huileuses, alcooliques ou oléoalcooliques, d'émulsions telles qu'une crème ou un lait, de gels oléoalcooliques, alcooliques ou hydroalcooliques, de bâtonnets solides, ou être conditionnée en aérosol.

Elle peut contenir les adjuvants cosmétiques habituellement utilisés dans ce type de composition tels que

des épaississants, des adoucissants, des humectants, des tensio-actifs, des conservateurs, des anti-mousses, des parfums, des huiles, des cires, de la lanoline, des propulseurs, des colorants et/ou pigments ayant pour fonction de colorer la composition elle-même ou la peau ou tout autre ingrédient habituellement utilisé en cosmétique.

Le composé de formule (1) ou (2) est présent dans des proportions en poids comprises entre 0,25 et 3% par rapport au poids total de la composition cosmétique protectrice de l'épiderme humain.

Comme solvant de solubilisation, on peut utiliser une huile, une cire et de façon générale tout corps gras, un monoalcool ou un polyol inférieur, un benzoate d'alcools en $C_{12}$-$C_{15}$ ou leurs mélanges. Les monoalcools ou polyols plus particulièrement préférés sont l'éthanol, l'isopropanol, le propylèneglycol, la glycérine et le sorbitol.

Une forme de réalisation de l'invention est une émulsion sous forme de crème ou de lait protecteurs comprenant en plus du composé de formule (1) ou (2), des alcools gras, des esters d'acides gras et notamment des triglycérides d'acides gras, des acides gras, de la lanoline, des huiles ou cires naturelles ou synthétiques et des émulsionnants, en présence d'eau.

Une autre forme de réalisation est constituée par des lotions huileuses à base d'huiles et cires naturelles ou synthétiques, de lanoline, et d'esters d'acides gras, notamment de triglycérides d'acides gras, ou par des lotions oléoalcooliques à base d'un alcool inférieur tel que l'éthanol ou d'un glycol tel que le propylèneglycol et/ou d'un polyol tel que la glycérine et d'huiles, de cires et d'esters d'acides gras tels que les triglycérides d'acides gras.

La composition cosmétique de l'invention peut également être un gel alcoolique comprenant un ou plusieurs alcools ou polyols inférieurs tels que l'éthanol, le propylèneglycol ou la glycérine et un épaississant tel que la silice. Les gels oléoalcooliques contiennent en outre une huile ou une cire naturelle ou synthétique.

Les bâtonnets solides sont constitués de cires et d'huiles naturelles ou synthétiques, d'alcools gras, d'esters d'acides gras, de lanoline et autres corps gras.

Dans le cas d'une composition conditionnée en aérosol, on utilise les propulseurs classiques tels que les alcanes, les fluoroalcanes et les chlorofluoroalcanes.

La présente invention vise également les compositions cosmétiques anti-solaires contenant au moins un composé de formule (1) ou (2) et pouvant contenir d'autres filtres UV-B et/ou UV-A.

Dans ce cas, le composé de formule (1) ou (2) est présent en des concentrations variant de 0,5 à 10% en poids et la quantité totale de filtres présents dans la composition anti-solaire, c'est-à-dire le composé de formule (1) ou (2) et éventuellement les autres filtres, est comprise entre 0,5 et 15% en poids par rapport au poids total de la composition anti-solaire.

Ces compositions anti-solaires se présentent sous les formes indiquées ci-dessus pour les compositions protectrices de l'épiderme humain.

Lorsque la composition cosmétique selon l'invention est destinée à protéger des rayons UV les cheveux naturels ou sensibilisés, cette composition peut se présenter sous forme de shampooing, de lotion, gel ou émulsion à rincer, à appliquer avant ou après le shampooing, avant ou après coloration ou décoloration, avant ou après permanente, de lotion ou gel coiffants ou traitants, de lotion ou gel pour le brushing ou la mise en plis, de spray de coiffage, de laque pour cheveux, de composition de permanente, de coloration ou décoloration des cheveux. Cette composition peut contenir, outre le composé de l'invention, divers adjuvants utilisés dans ce type de composition, tels que des agents tensio-actifs, des épaississants, des polymères, des adoucissants, des conservateurs, des stabilisateurs de mousse, des électrolytes, des solvants organiques, des dérivés siliconés, des huiles, des cires, des agents anti-gras, des colorants et/ou pigments ayant pour fonction de colorer la composition elle-même ou la chevelure ou tout autre ingrédient habituellement utilisé dans le domaine capillaire.

Elle contient 0,25 à 5% en poids de composé de formule (1) ou (2).

La présente invention vise également les compositions cosmétiques renfermant un constituant sensible à la lumière et contenant au moins un composé de formule (1) ou (2) à titre d'agent de protection contre les rayons ultraviolets. Ces compositions cosmétiques sont constituées par des compositions capillaires telles que les laques pour cheveux, les lotions de mise en plis éventuellement traitantes ou démêlantes, les shampooings colorants, les compositions tinctoriales pour cheveux, par des produits de maquillage tels que les vernis à ongles, les crèmes et huiles de traitement pour l'épiderme, les fonds de teint, les bâtons de rouge à lèvre, les compositions pour les soins de la peau telles que des huiles ou crèmes pour le bain, ainsi que toute autre composition cosmétique pouvant présenter du fait de ses constituants, des problèmes de stabilité à la lumière au cours du stockage.

De telles compositions contiennent 0,25 à 3% en poids de composé de formule (1) ou (2).

L'invention vise également un procédé de protection des compositions cosmétiques contre les rayons ultraviolets, consistant à incorporer à ces compositions une quantité efficace d'au moins un composé de formule

(1) ou (2).

Les exemples ci-après illustrent l'invention sans en limiter la portée.

Exemple de référence 1 :

Dans un ballon tricol maintenu à 96°C par un bain d'huile, muni d'une agitation centrale et d'un réfrigérant ascendant, on charge 110,8 g (0,374 mole) d'allyloxy-4' benzylidène-3 camphre, préparé conformément à l'exemple 8 de FR-A-2430938, 160 g de toluène et 12 µl d'une solution dans l'hexane (à 8,45% en poids de platine métal) d'un complexe de platine préparé à partir d'acide chloroplatinique et de divinyl-1,3 tétraméthyl-1,1,3,3 disiloxane comme décrit dans le brevet US-A-3814730.

On ajoute en une demi-heure 50 g d'un polymère statistique à SiH de formule :

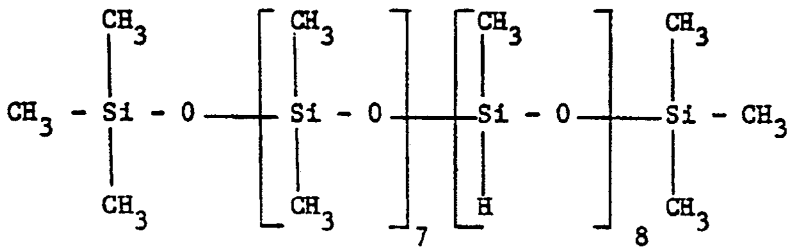 (1)

titrant 731,3 meq/100 g en fonction SiH (meq = milliéquivalent).

On vérifie au bout de 10 heures de réaction, par dosage des SiH résiduels au moyen de potasse butanolique, que le taux de transformation des fonctions SiH est de 99%.

On obtient alors une huile limpide, légèrement jaune, de très forte viscosité, après avoir éliminé le toluène par distillation à 100°C, sous pression réduite de 0,66 kPa.

Sur un échantillon de l'huile obtenue, on effectue une analyse de Résonance Magnétique Nucléaire de proton (RMN$^1$H) à 360 MHz dans $CDCl_3$. Le spectre RMN est conforme à la structure attendue.

Le taux de dérivé de camphre de départ dans l'huile obtenue après élimination du solvant est de 6,4% en poids.

L'élimination de ce dérivé est aisément effectuée par passage de l'huile sur une colonne de gel de silice (support Kieselgel ART 7754, commercialisé par la Société MERCK) avec, comme solvant d'élution du dérivé organique, un mélange 95/5 en volume d'hexane/acétate de butyle et comme solvant d'élution de l'huile, l'acétate de butyle.

L'huile purifiée contient moins de $3.10^{-3}$% en poids de dérivé de camphre de départ. Elle présente un indice de réfraction d'environ 1,542 et le spectre UV dans le chloroforme donne les résultats suivants :

$$\lambda_{max} = 316 \text{ nm}$$

$$E\,(1\%) = 576,$$

E étant la densité optique mesurée à la longueur d'onde du maximum d'absorption $\lambda_{max}$ pour une solution contenant 1% en poids de produit filtrant.

Exemple de référence 2

Préparation de l'allyloxy-4' méthoxy-3' benzylidène-3 camphre.

On répète exactement le mode opératoire de l'exemple 8 du brevet français FR-A-2430938 sauf que l'on utilise comme produit de départ l'hydroxy-4' méthoxy-3' benzylidène-3 camphre. On obtient le produit désiré qui présente un point de fusion de 75°C.

On effectue ensuite exactement les mêmes opérations qu'à l'exemple 1 sauf que l'on utilise :

- 30,6 g d'allyloxy-4' méthoxy-3' benzylidène-3 camphre,
- 31 g de toluène,
- 3,4 µl de solution catalysante au platine,
- 12,5 g de polymère à SiH de formule :

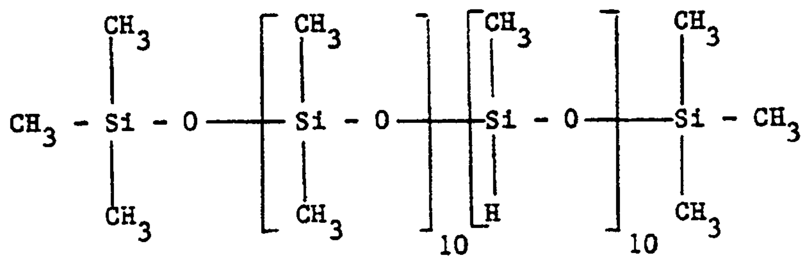

(1)

titrant 733,9 meq/100 g en fonction SiH (meq = milliéquivalent).

On obtient un taux de transformation des SiH de 99% après 9 heures de réaction.

Après élimination du toluène, on isole 40,0 g d'huile légèrement jaune très visqueuse titrant 5,3% en poids de dérivé de camphre de départ.

L'analyse RMN$^1$H confirme la structure attendue.

L'élimination du dérivé de camphre est effectuée de la même façon qu'à l'exemple 1 sauf que le solvant d'élution du dérivé de camphre est le dichlorométhane.

L'huile purifiée contient moins de $3.10^{-3}$% en poids de dérivé de camphre et présente un indice de réfraction d'environ 1,540.

Le spectre UV dans le chloroforme donne les résultats suivants :

$$\lambda_{max} = 327 \text{ nm}$$

$$E\,(1\%) = 416$$

Exemple de référence 3

3a — préparation de l'allyl-3′ méthoxy-4′ benzylidène-3 camphre

1er stade

Préparation de l'allyl-3′ hydroxy-4′ benzylidène-3 camphre

On chauffe pendant 24 heures sous agitation à 185°C, 296 g (1 mole) d'allyloxy-4′ benzylidène-3 camphre obtenu conformément à l'exemple 8 de FR-A 2430938.

Le mélange réactionnel refroidi est recristallisé dans l'éther éthylique. On obtient ainsi 270 g d'allyl-3′ hydroxy-4′ benzylidène-3 camphre possédant les caractéristiques suivantes :

— point de fusion : 150°C

— spectre RMN $^1$H ($CDCl_3$) : spectre conforme à la structure attendue

- spectre UV (éthanol) $\lambda_{max}$ : 327 nm
$\varepsilon$ : 22600

— analyse élémentaire

| | |
|---|---|
| calculé % : | C 81,04 H 8,16 |
| trouvé % : | C 81,11 H 8,18 |

2ème stade

Préparation de l'allyl-3′ méthoxy-4′ benzylidène-3 camphre

On dissout 9 g (0,03 mole) d'allyl-3′ hydroxy-4′ benzylidène-3 camphre obtenu au premier stade ci-dessus dans 150 cm$^3$ de diméthoxy-1,2-éthane préalablement séché sur tamis moléculaire. On ajoute lentement 2,56 g d'hydrure de sodium et on chauffe à 60°C. On introduit goutte à goutte 8,52 g (0,06 mole) d'iodure de méthyle,

puis on porte au reflux pendant 2 heures. Le solvant est évaporé et le résidu est repris par 50 $cm^3$ d'éther éthylique. La phase éthérée est lavée 2 fois à l'eau, puis séchée sur sulfate de sodium. Après évaporation du solvant, on récupère 8,8 g d'allyl-3' méthoxy-4' benzylidène-3 camphre, sous forme de cristaux blancs possédant les caractéristiques suivantes :

— point de fusion : 37°C

— spectre de $RMN^1H$ ($CDCl_3$) : spectre conforme à la structure attendue

- spectre UV (éthanol à 95°) $\lambda_{max}$ : 322 nm
$\varepsilon$ : 23600

Analyse élémentaire :

| | |
|---|---|
| Calculé % : | C 81,25 H 8,44 0 10,31 |
| trouvé % : | C 81,35 H 8,60 0 10,50 |

3b — préparation du polydiméthylsiloxane de formule

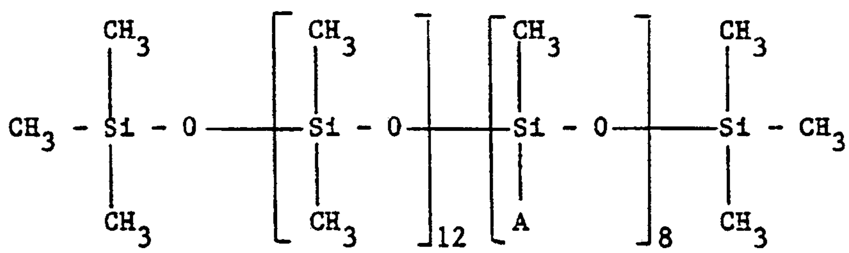
(1)

où A est le radical organique de formule :

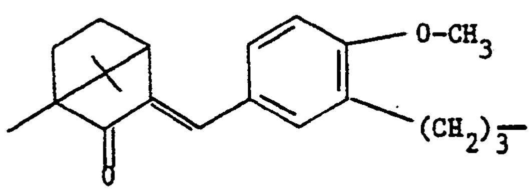

Dans un ballon tricol maintenu à 100°C par un bain d'huile, muni d'une agitation centrale et d'un réfrigérant ascendant, on charge 25 g (80,53 mmoles) d'allyl-3' méthoxy-4' benzylidène-3 camphre et 25 g de toluène.

Lorsque la température du milieu réactionnel est de 100°C, on introduit 1,60 microlitre d'une solution dans l'hexane (à 9,92% en poids de platine métal) du complexe de platine utilisé à l'exemple 1 ci-dessus.

On ajoute alors en 1 heure 30 minutes 10,37 g d'un polymère statistique à SiH de formule :

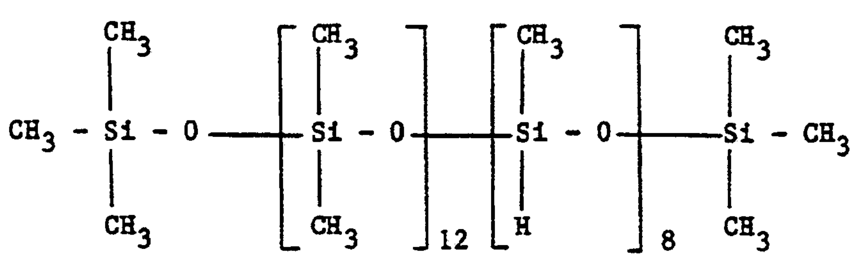
(1)

titrant 705,8 meq/100 g en fonction SiH.

Le taux de transformation des SiH est quantitatif après 11 heures de réaction.

Après élimination du toluène, on isole une huile jaune visqueuse titrant 11,7% en poids de dérivé du camphre de départ.

L'analyse RMN¹H et l'analyse RMN²⁹ Si à 49,7 MHz dans $CDCl_3$ + Fe $(Acac)_3$ indiquent que le produit est conforme à la structure attendue.

L'élimination du dérivé de camphre de départ est effectuée de la même façon qu'à l'exemple 1.

L'huile purifiée contient moins de $3.10^{-3}$% en poids de dérivé de camphre de départ.

Le spectre UV dans le chloroforme montre que :

$$\lambda_{max} = 322 \text{ nm}$$

EXEMPLE 1

On prépare une crème solaire de composition suivante :

| | | |
|---|---|---|
| - Polydiméthylsiloxane à greffons allyloxy-4' benzylidène-3 camphre de l'exemple de référence 1 | | 5 g |
| - Mélange d'alcools cétylstéarylique (80%) et cétylstéarylique oxyéthyléné à 33 moles d'oxyde d'éthylène (20%) vendu par la Société HENKEL sous la dénomination "SINNOWAX AO" | | 7 g |
| - Monostéarate de glycérol | | 2 g |
| - Alcool cétylique | | 1,5 g |
| - Benzoate d'alcools en $C_{12}$-$C_{15}$ vendu par la Société WITCO sous la dénomination "FINSOLV TN" | | 15 g |
| - Glycérine | | 20 g |
| - Polydiméthylsiloxane | | 1,5 g |
| - Parfum, conservateur | qs | |
| - Eau | qsp | 100 g |

Cette crème est préparée selon les techniques classiques de préparation d'émulsions en dissolvant le filtre dans la phase grasse contenant les émulsionnants, en chauffant cette phase grasse aux alentours de 70-80°C et en ajoutant, sous vive agitation, l'eau chauffée à la même température ; on maintient l'agitation pendant 10 à 15 minutes, puis on laisse refroidir sous agitation modérée et vers 40°C, on ajoute parfum et conservateur.

EXEMPLE 2

On prépare de la même façon que dans l'exemple 1 une crème solaire de composition suivante :

| | | | |
|---|---|---|---|
| - Polydiméthylsiloxane à greffons allyloxy-4' benzylidène-3 camphre de l'exemple de référence 1 | | 3,5 | g |
| - Lanoline liquide | | 7 | g |
| - Triglycéride d'acides gras en $C_{10}$ à $C_{18}$ vendu sous la dénomination "NESATOL" par la Société VEVY | | 5 | g |
| - Ester d'acide oléique polyéthoxylé et de glycérol vendu sous la dénomination "LABRAFIL M 1969 CS" par la Société GATTEFOSSE | | 2,5 | g |
| - Mélange de stéarate de glycérol et de stéarate de polyéthylène glycol à 100 moles d'oxyde d'éthylène, vendu sous la dénomination "ARLACEL 165" par la Société ICI | | 5 | g |
| - Alcool stéarylique | | 1 | g |
| - Acide stéarique | | 2,5 | g |
| - Benzoate d'alcools en $C_{12}$-$C_{15}$ vendu sous la dénomination "FINSOLV TN" par la Société WITCO | | 9 | g |
| - Mélange de phosphate de cétyle et de phosphate de cétyle/diéthanolamine vendu sous la dénomination "AMPHISOL" par la Société GIVAUDAN | | 0,5 | g |
| - Triéthanolamine | | 0,2 | g |
| - Conservateur, parfum | qs | | |
| - Eau | qsp | 100 | g |

EXEMPLE 3

On prépare un après-shampooing de composition suivante :

| | | | | |
|---|---|---|---|---|
| - Polydiméthylsiloxane à greffons allyloxy-4' benzylidène-3 camphre de l'exemple de référence 1 | | | 2 | g |
| - Mélange d'alcools cétylstéarylique (80%) et cétylstéarylique oxyéthyléné à 33 moles d'oxyde d'éthylène (20%) vendu par la Société HENKEL sous la dénomination "SINNOWAX AO" | | | 3 | g |
| - Alcool cétylique | | | 1 | g |
| - Alcool stéarylique | | | 1 | g |
| - Triéthanolamine | qs | pH : 7,3 | | |
| - Eau | qsp | | 100 | g |

Les alcools cétylique et stéarylique et le Sinnowax AO sont fondus vers 60-70°C ; à cette température on dissout le polydiméthylsiloxane à greffons allyloxy-4' benzylidène-3 camphre. On laisse le mélange revenir à température ambiante et on le dilue alors avec l'eau, on ajoute la triéthanolamine pour ajuster le pH à 7,3.

EXEMPLE 4

On prépare un spray de protection capillaire de composition suivante :

| | | | |
|---|---|---|---|
| - Polydiméthylsiloxane à greffons allyloxy-4' benzylidène-3 camphre de l'exemple de référence 1 | | 2 | g |
| - Benzoate d'alcools en $C_{12}$-$C_{15}$ vendu par la société WITCO sous la dénomination "FINSOLV TN" | | 12 | g |
| - Alcool éthylique | qsp | 100 | g |

La composition ci-dessus est conditionnée en flacon pompe.

On dissout à température ambiante le polydiméthylsiloxane à greffons allyloxy-4' benzylidène-3 camphre dans le Finsolv TN ; on complète à 100 g par addition de l'alcool éthylique.

EXEMPLE 5

On prépare un lait solaire (émulsion H/E) de composition suivante :

| | | |
|---|---|---|
| - Mélange alcool cétylique/alcool stéarylique (30/70) | 2,4 | g |
| - Mélange d'alcool cétylique/alcool stéarylique oxyéthyléné à 33 moles d'oxyde d'éthylène (30/70) | 0,6 | g |
| - Mélange de mono et distéarate de glycérol non autoémulsionnable | 1 | g |
| - Huile de vaseline | 6 | g |
| - Myristate d'isopropyle | 3 | g |
| - Silicone DC 200-350, CST vendu par la Société DOW CORNING | 1 | g |
| - Alcool cétylique pur | 1 | g |
| - Alcool stéarylique pur | 2 | g |
| - Polydiméthylsiloxane à greffons allyloxy-4' méthoxy-3' benzylidène-3 camphre de l'exemple de référence 2 | 0,5 | g |
| - p-méthoxycinnamate de 2-éthylhexyle | 2 | g |

Phase aqueuse

| | | | |
|---|---|---|---|
| - Glycérine | | 20 | g |
| - conservateurs | qs | | |
| - Parfum | qs | | |
| - Eau | qsp | 100 | g |

Ce lait est préparé en ajoutant la phase grasse portée à 80°C à la phase aqueuse chauffée à la même température sous agitation.

EXEMPLE 6

On prépare un gel protecteur pour la peau et les cheveux de composition suivante :

| | | | |
|---|---|---|---|
| - Polydiméthylsiloxane à greffons allyloxy-4' méthoxy-3' benzylidène-3 camphre de l'exemple de référence 2 | | | 0,5 g |
| - Monobutyléther du diéthylène glycol | | | 10 g |
| - Acide polyacylique réticulé, PM = 4 000 000, vendu par la Société GOODRICH sous la dénomination "CARBOPOL 940" | | | 1 g |
| - Eau | qsp | | 100 g |
| - Triéthanolamine | qs | pH : 6,8 | |

EXEMPLE 7

On prépare une crème pour les soins de la peau de composition suivante :

| | | | |
|---|---|---|---|
| - Polydiméthylsiloxane à greffons allyloxy-4' méthoxy-3' benzylidène-3 camphre de l'exemple de référence 2 | | | 1 g |
| - Monobutyléther du diéthylène glycol | | | 10 g |
| - Mélange d'alcool cétylstéarylique et d'alcool cétylstéarylique oxyéthylené à 33 moles d'oxyde d'éthylène vendu sous la dénomination "SINNOWAX AO" par la Société HENKEL | | | 2 g |
| - Alcool stéarylique pur | | | 1 g |
| - Alcool cétylique pur | | | 1 g |
| - Gomme de xanthane vendue par la Société KELCO sous la dénomination "KELTROL T" | | | 0,7 g |
| - Eau | qsp | | 100 g |
| - HCl | qs | pH : 5,8 | |

EXEMPLE 8

On prépare un gel protecteur pour la peau et les cheveux analogue à celui de l'exemple 6, en utilisant 0,5 g de polydiméthylsiloxane à greffons allyl-3' méthoxy-4' benzylidène-3 camphre de l'exemple de référence 3.

EXEMPLE 9

Lotion traitante pour les cheveux

| | | |
|---|---|---|
| - Polydiméthylsiloxane à greffons allyloxy-4' benzylidène-3 camphre de l'exemple de référence 1 | 5 | g |
| - Benzoate d'alcools $C_{12}$-$C_{15}$ vendu sous la dénomination de "FINSOLV TN" par la Société WITCO | 65 | g |
| - Isoparaffine vendue sous le nom d'"ISOPAR H" par la Société EXXON CHEMICALS | 35 | g |

Cette lotion limpide facilite le démêlage des cheveux mouillés et des cheveux séchés tout en les protégeant du rayonnement ultra-violet.

**Revendications**

1. Utilisation en cosmétique de diorganopolysiloxanes à fonction benzylidène-3 camphre choisis parmi ceux de formule :

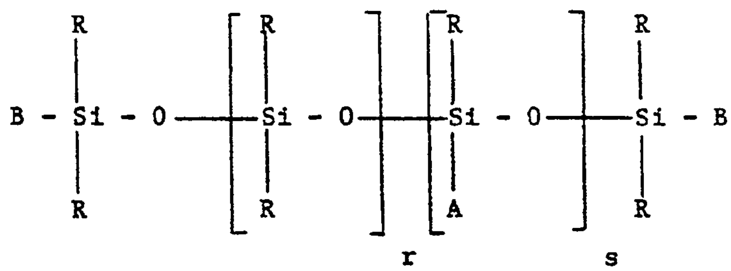

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre entier choisi entre 0 et 200 inclusivement,

s est un nombre entier choisi entre 0 et 50 inclusivement et si s est 0, au moins l'un des deux symboles B désigne A, et ceux de formule :

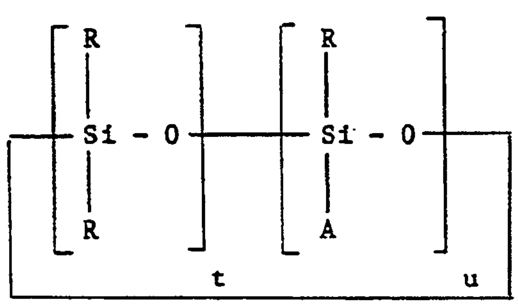

(2)

dans laquelle

R a la même signification qu'à la formule (1),

u est un nombre entier compris entre 1 et 20 inclus et

t est un nombre entier compris entre 0 et 20 inclus,

t + u est égal ou supérieur à 3,

formules dans lesquelles le symbole A est un radical de formule :

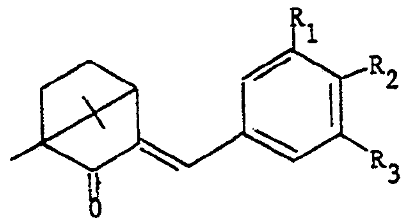

(3)

dans laquelle :

$R_1$ et $R_2$ représentent un atome d'hydrogène, un radical alkyle en $C_1$-$C_6$, un radical alcoxy en $C_1$-$C_6$, hydroxyle, triméthylsilyloxy ou un radical divalent Y de formule :

$$-(O)_n-(CH_2)_p-\underset{R_4}{\underset{|}{C}}H-CH_2- \quad ,$$

l'un des deux radicaux $R_1$ et $R_2$ représentant nécessairement le radical divalent Y,

$R_4$ représente un atome d'hydrogène ou un radical alkyle en $C_1$-$C_4$,

n est 0 ou 1,

p est un nombre entier compris entre 1 et 10 inclus,

$R_3$ représente un atome d'hydrogène, un radical alkyle en $C_1$-$C_6$ ou un alcoxy en $C_1$-$C_6$.

2. Utilisation en cosmétique d'un polymère statistique ou à blocs de formule (1) ou (2) selon la revendication 1, présentant au moins l'une des caractéristiques suivantes :

— R est méthyle,
— B est méthyle,
— r est compris entre 5 et 20 inclus,
— s est compris entre 2 et 15 inclus,
— t + u est compris entre 3 et 10 inclus,
— $R_1$ est H ou méthoxy,
— $R_2$ est H ou méthoxy,

l'un des deux radicaux $R_1$ ou $R_2$ représentant le radical divalent Y dans lequel n = 0 ou 1, p = 1, $R_4$ = H ou méthyle,

— $R_3$ est H ou méthoxy.

3. Utilisation en cosmétique à titre d'agents filtrant le rayonnement UV de longueurs d'onde comprises entre 280 et 360 nm, de diorganopolysiloxanes à fonction benzylidène-3 camphre choisis parmi ceux de formule :

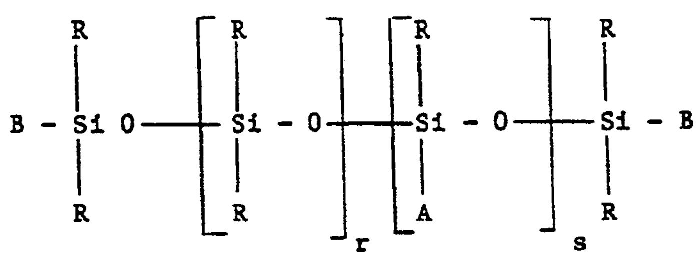

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre entier choisi entre 0 et 200 inclusivement,

s est un nombre entier choisi entre 0 et 50 inclusivement et si s est 0, au moins l'un des deux symboles B désigne A, et ceux de formule :

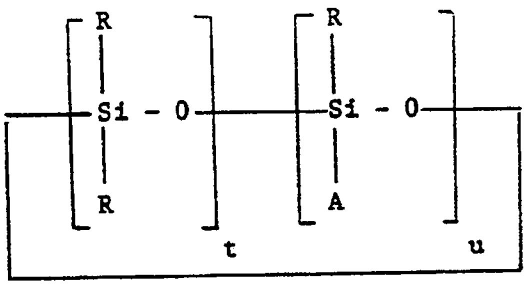

(2)

dans laquelle

R a la même signification qu'à la formule (1),

u est un nombre entier compris entre 1 et 20 inclus et

t est un nombre entier compris entre 0 et 20 inclus,

t + u est égal ou supérieur à 3, formules dans lesquelles le symbole A est un radical de formule :

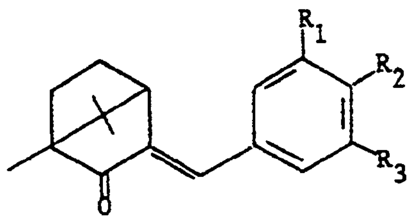

(3)

dans laquelle :

$R_1$ et $R_2$ représentent un atome d'hydrogène, un radical alkyle en $C_1$-$C_6$, un radical alcoxy en $C_1$-$C_6$, hydroxyle, triméthylsilyloxy ou un radical divalent Y de formule :

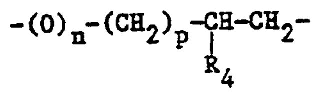

,

l'un des deux radicaux $R_1$ et $R_2$ représentant nécessairement le radical divalent Y,

$R_4$ représente un atome d'hydrogène ou un radical alkyle en $C_1$-$C_4$,

n = 0 ou 1

p est un nombre entier compris entre 1 et 10 inclus,

$R_3$ représente un atome d'hydrogène, un radical alkyle en $C_1$-$C_6$ ou un alcoxy en $C_1$-$C_6$.

4. Utilisation en cosmétique à titre d'agents filtrant le rayonnement UV de longueurs d'onde comprises entre 280 et 360 nm, de diorganopolysiloxanes statistiques ou à blocs de formules (1) ou (2) selon la revendication 3, présentant au moins l'une des caractéristiques suivantes :

— R est méthyle,

— B est méthyle,

— r est compris entre 5 et 20 inclus,

— s est compris entre 2 et 15 inclus,

— t + u est compris entre 3 et 10 inclus,

— $R_1$ est H ou méthoxy,

— $R_2$ est H ou méthoxy,

l'un des deux radicaux $R_1$ ou $R_2$ représentant le radical divalent Y dans lequel n est égal à 0 ou 1, p est égal à 1, $R_4$ est H ou méthyle, et $R_3$ est H ou méthoxy.

5. Utilisation en cosmétique d'un polydiméthylsiloxane à greffons allyloxy-4′ benzylidène-3 camphre de formule (1) selon la revendication 1 dans laquelle R et B désignent méthyle, r est égal à 7 et s est égal à 8.

6. Utilisation en cosmétique d'un polydiméthylsiloxane à greffons allyloxy-4′ méthoxy-3′ benzylidène-3 camphre de formule (1) selon la revendication 1 dans laquelle R et B désignent méthyle, r est égal à 10 et s est égal à 10.

7. Utilisation en cosmétique d'un polydiméthylsiloxane à greffons allyl-3′ méthoxy-4′ benzylidène-3 camphre de formule (1) selon la revendication 1 dans laquelle R et B désignent méthyle, r est égal à 12 et s est

égal à 8.

8. Composition cosmétique, caractérisée par le fait qu'elle comprend, dans un support cosmétiquement acceptable, entre 0,25 et 15% par rapport au poids total de la composition d'au moins un diorganopolysiloxane à fonction benzylidène-3 camphre choisi parmi ceux de formule :

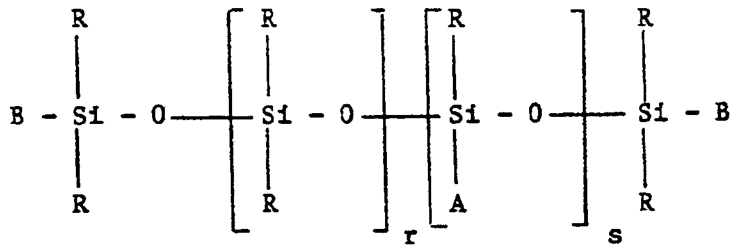

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre entier choisi entre 0 et 200 inclusivement,

s est un nombre entier choisi entre 0 et 50 inclusivement et si s est 0, au moins l'un des deux symboles B désigne A, et ceux de formule :

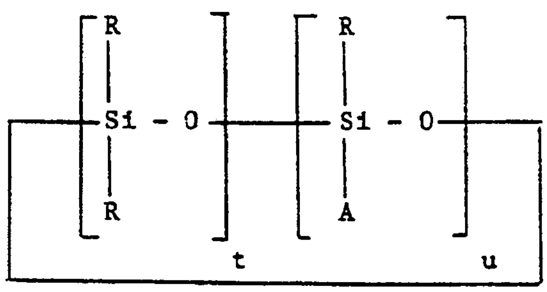

(2)

dans laquelle

R a la même signification qu'à la formule (1),

u est un nombre entier compris entre 1 et 20 inclus et

t est un nombre entier compris entre 0 et 20 inclus,

t + u est égal ou supérieur à 3, formules dans lesquelles le symbole A est un radical de formule :

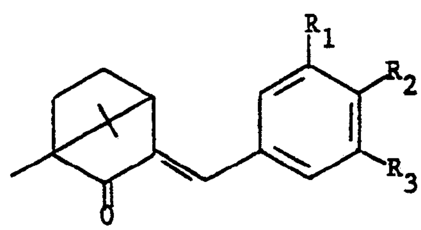

(3)

dans laquelle :

$R_1$ et $R_2$ représentent un atome d'hydrogène, un radical alkyle en $C_1$-$C_6$, un radical alcoxy en $C_1$-$C_6$, hydroxyle, triméthylsilyloxy ou un radical divalent Y de formule :

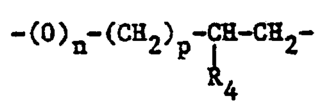 ,

l'un des deux radicaux $R_1$ et $R_2$ représentant nécessairement le radical divalent Y,
$R_4$ représente un atome d'hydrogène ou un radical alkyle en $C_1$-$C_4$,
n = 0 ou 1,
p est un nombre entier compris entre 1 et 10 inclus,
$R_3$ représente un atome d'hydrogène, un radical alkyle en $C_1$-$C_6$ ou un alcoxy en $C_1$-$C_6$.

9. Composition cosmétique selon la revendication 8, caractérisée par le fait qu'elle comprend un diorganopolysiloxane à fonction benzylidène-3 camphre de formule (1) ou (2), statistique ou à blocs, présentant au moins l'une des caractéristiques suivantes : R est méthyle, B est méthyle, r est compris entre 5 et 20 inclus, s est compris entre 2 et 15 inclus, t + u est compris entre 3 et 10 inclus, $R_1$ est H ou méthoxy, $R_2$ est H ou méthoxy, l'un des deux radicaux $R_1$ ou $R_2$ représentant un radical divalent Y dans lequel n est égal à 0 ou 1, p est égal à 1, $R_4$ est H ou méthyle, et $R_3$ est H ou méthoxy.

10. Composition cosmétique selon la revendication 8 ou 9, caractérisée par le fait qu'elle comprend un polydiméthylsiloxane à greffons allyloxy-4' benzylidène-3 camphre de formule (1) dans laquelle R et B désignent le groupe méthyle, r est égal à 7 et s est égal à 8.

11. Composition cosmétique selon la revendication 8 ou 9, caractérisée par le fait qu'elle comprend un polydiméthylsiloxane à greffons allyloxy-4' méthoxy-3' benzylidène-3 camphre de formule (1) dans laquelle R et B désignent le groupe méthyle, r est égal à 10 et s est égal à 10.

12. Composition cosmétique selon la revendication 8 ou 9, caractérisée par le fait qu'elle comprend un polydiméthylsiloxane à greffons allyl-3' méthoxy-4' benzylidène-3 camphre de formule (1) dans laquelle R et B désignent le groupe méthyle, r est égal à 12 et s est égal à 8.

13. Composition cosmétique selon l'une quelconque des revendications 8 à 12, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, monoalcools et polyols inférieurs, benzoates d'alcools en $C_{12}$-$C_{15}$, propulseurs, colorants et pigments.

14. Composition cosmétique selon l'une quelconque des revendications 8 à 13, caractérisée par le fait qu'elle se présente sous forme de lotion huileuse, alcoolique ou oléoalcoolique, d'émulsion, gel oléoalcoolique, alcoolique ou hydroalcoolique, bâtonnet solide, spray ou aérosol.

15. Composition cosmétique selon l'une quelconque des revendications 8 à 14, caractérisée par le fait qu'elle constitue une composition protectrice de l'épiderme humain et contient 0,25 à 3% en poids de diorganopolysiloxane de formule (1) ou (2).

16. Composition cosmétique selon l'une quelconque des revendications 8 à 14, se présentant sous forme de composition anti-solaire, caractérisée par le fait qu'elle contient 0,5 à 15% en poids de diorganopolysiloxane de formule (1) ou (2).

17. Composition cosmétique anti-solaire selon la revendication 16, caractérisée par le fait qu'elle contient en outre un agent filtrant les rayons UV-B et/ou UV-A, la quantité totale de filtres étant comprise entre 0,5 et 15% en poids par rapport au poids total de la composition.

18. Composition cosmétique selon l'une quelconque des revendications 8 à 13, destinée à être appliquée sur les cheveux, caractérisée par le fait qu'elle se présente sous forme de shampooing, lotion, gel ou émulsion à rincer, à appliquer avant ou après le shampooing, avant ou après coloration ou décoloration, avant ou après permanente, lotion ou gel coiffants ou traitants, lotion ou gel pour le brushing ou la mise en plis, spray de coiffage, laque pour cheveux, composition de permanente, de décoloration ou de coloration et comprend 0,25 à 5% en poids de diorganopolysiloxane de formule (1) ou (2).

19. Composition cosmétique selon l'une quelconque des revendications 8 à 13, se présentant sous forme d'une composition cosmétique colorée ou non, caractérisée par le fait qu'elle est constituée par une composition capillaire, un produit de maquillage ou une composition pour les soins ou le traitement de la peau, comprenant 0,25 à 3% en poids de diorganopolysiloxane de formule (1) ou (2).

20. Procédé de traitement cosmétique de la peau et des cheveux naturels ou sensibilisés en vue de les protéger contre le rayonnement ultraviolet, caractérisé par le fait qu'il consiste à appliquer sur la peau ou les cheveux une quantité efficace d'une composition cosmétique contenant au moins un diorganopolysiloxane à fonction benzylidène-3 camphre de formule (1) ou (2) défini dans l'une quelconque des revendications 3 à 6.

21. Procédé de protection contre les rayons ultraviolets, d'une composition cosmétique renfermant un constituant sensible à la lumière caractérisé par le fait qu'il consiste à incorporer à cette composition au moins 0,25 à 3% en poids d'un diorganopolysiloxane à fonction benzylidène-3 camphre de formule (1) ou (2) définie dans l'une quelconque des revendications 3 à 6.

**Revendications pour l'Etat contractant suivant : ES**

1. Procédé de préparation d'une composition cosmétique sous forme de lotion huileuse, alcoolique ou

oléoalcoolique, d'émulsion, de gel oléoalcoolique, alcoolique ou hydroalcoolique, de bâtonnet solide, éventuellement conditionnée en aérosol, pour la protection de la peau et des cheveux contre les rayonnements ultraviolets, caractérisé par le fait que l'on introduit dans un milieu cosmétiquement acceptable, dans des proportions comprises entre 0,25 et 15% par rapport au poids total de la composition au moins un diorganopolysiloxane à fonction benzylidène-3 camphre choisi parmi ceux de formule :

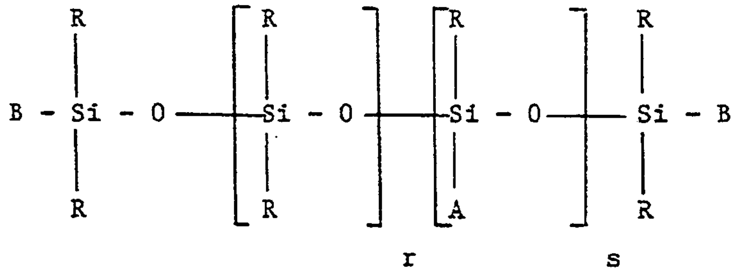

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre entier choisi entre 0 et 200 inclusivement,

s est un nombre entier choisi entre 0 et 50 inclusivement et si s est 0, au moins l'un des deux symboles B désigne A, et ceux de formule :

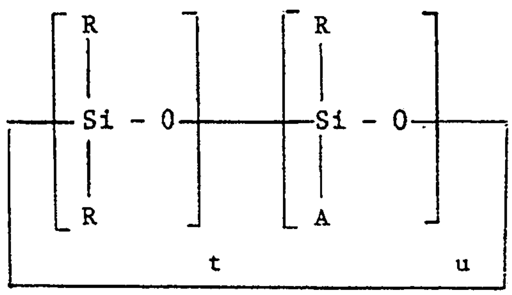

(2)

dans laquelle

R a la même signification qu'à la formule (1),

u est un nombre entier compris entre 1 et 20 inclus et

t est un nombre entier compris entre 0 et 20 inclus,

t + u est égal ou supérieur à 3, formules dans lesquelles le symbole A est un radical de formule :

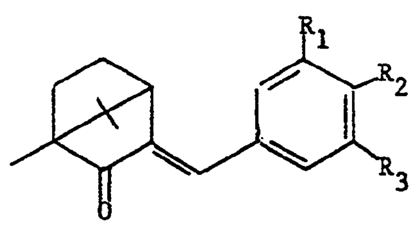

(3)

dans laquelle :

$R_1$ et $R_2$ représentent un atome d'hydrogène, un radical alkyle en $C_1$-$C_6$ un radical alcoxy en $C_1$-$C_6$ hydroxyle, triméthylsilyloxy ou un radical divalent Y de formule :

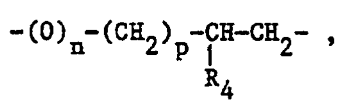

l'un des deux radicaux $R_1$ et $R_2$ représentant nécessairement le radical divalent Y,

$R_4$ représente un atome d'hydrogène ou un radical alkyle en $C_1$-$C_4$,

n est 0 ou 1,

p est un nombre entier compris entre 1 et 10 inclus,

$R_3$ représente un atome d'hydrogène, un radical allyle en $C_1$-$C_8$ ou un alcoxy en $C_1$-$C_8$.

2. Procédé selon la revendication 1, caractérisé par le fait que l'on utilise un polymère statistique ou à blocs de formule (1) ou (2) présentant au moins l'une des caractéristiques suivantes :

— R est méthyle,

— B est méthyle,

— r est compris entre 5 et 20 inclus,

— s est compris entre 2 et 15 inclus,

— t + u est compris entre 3 et 10 inclus,

— $R_1$ est H ou méthoxy,

— $R_2$ est H ou méthoxy,

l'un des deux radicaux $R_1$ ou $R_2$ représentant le radical divalent Y dans lequel n = 0 ou 1, p = 1, $R_4$ = H ou méthyle,

— $R_3$ est H ou méthoxy.

3. Procédé selon la revendication 1, caractérisé par le fait que l'on utilise un polydiméthylsiloxane à greffons allyloxy-4' benzylidène-3 camphre de formule (1) dans laquelle R et B désignent méthyle, r est égal à 7 et s est égal à 8.

4. Procédé selon la revendication 1, caractérisé par le fait que l'on utilise un polydiméthylsiloxane à greffons allyloxy-4' méthoxy-3, benzylidène-3 camphre de formule (1) dans laquelle R et B désignent méthyle, r est égal à 10 et s est égal à 10.

5. Procédé selon la revendication 1, caractérisé par le fait qu'on utilise un polydiméthyls iloxane à greffons allyl-3' méthoxy-4' benzylidène-3 camphre de formule (1) dans laquelle R et B désignent méthyle, r est égal à 12 et s est égal à 8.

6. Composition cosmétique destinée à la protection de la peau ou des cheveux contre les rayonnements ultraviolets, caractérisée par le fait qu'elle comprend, dans un support cosmétiquement acceptable, au moins dans une quantité comprise entre 0,25 à 15% en poids par rapport au poids total de la composition, un diorganopolysiloxane à fonction benzylidène-3 camphre choisi parmi ceux de formule :

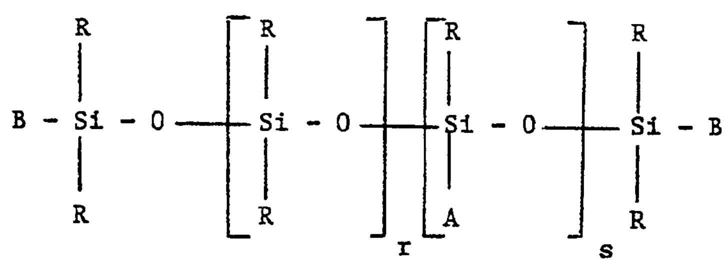

(1)

dans laquelle les symboles :

R, identiques ou différents, sont choisis parmi les radicaux alkyle en $C_1$-$C_{10}$, phényle et trifluoro-3,3,3 propyle, au moins 80% en nombre des radicaux R étant des radicaux méthyle,

B, identiques ou différents, sont choisis parmi les radicaux R et le radical A,

r est un nombre entier choisi entre 0 et 200 inclusivement,

$R_3$ représente un atome d'hydrogène, un radical alkyle en $C_1$-$C_8$ ou un alcoxy en $C_1$-$C_8$.

7. Composition cosmétique selon la revendication 6, caractérisée par le fait qu'elle comprend un diorganopolysiloxane à fonction benzylidène-3 camphre de formule (1) ou (2), statistique ou à blocs, présentant au moins l'une des caractéristiques suivantes : R est méthyle, B est méthyle, r est compris entre 5 et 20 inclus, s est compris entre 2 et 15 inclus, t + u est compris entre 3 et 10 inclus, $R_1$ est H ou méthoxy, $R_2$ est H ou méthoxy, l'un des deux radicaux $R_1$ ou $R_2$ représentant un radical divalent Y dans lequel n est égal à 0 ou 1, p est égal à 1, $R_4$ est H ou méthyle, et $R^3$ est H ou méthoxy.

8. Composition cosmétique selon la revendication 6 ou 7, caractérisée par le fait qu'elle comprend un polydiméthylsiloxane à greffons allyloxy-4' benzylidène-3 camphre de formule (1) dans laquelle R et B désignent le groupe méthyle, r est égal à 7 et s est égal à 8.

9. Composition cosmétique selon la revendication 6 ou 7, caractérisée par le fait qu'elle comprend un polydiméthylsiloxane à greffons allyloxy-4' méthoxy-3' benzylidène-3 camphre de formule (1) dans laquelle R et B

désignent le groupe méthyle, r est égal à 10 et s est égal à 10.

10. Composition cosmétique selon la revendication 6 ou 7, caractérisée par le fait qu'elle comprend un polydiméthylsiloxane à greffons allyl-3′ méthoxy-4′ benzylidène-3 camphre de formule (1) dans laquelle R et B désignent le groupe méthyle, r est égal à 12 et s est égal à 8.

11. Composition cosmétique selon l'une quelconque des revendications 6 à 10, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, monoalcools et polyols inférieurs, benzoates d'alcools en $C_{12}$-$C_{15}$, propulseurs, colorants et pigments.

12. Composition cosmétique selon l'une quelconque des revendications 6 à 11, caractérisée par le fait qu'elle se présente sous forme de lotion huileuse, alcoolique ou oléoalcoolique, d'émulsion, s est un nombre entier choisi entre 0 et 50 inclusivement et si s est 0, au moins l'un des deux symboles B désigne A, et ceux de formule :

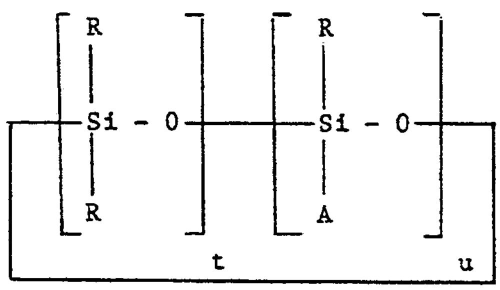

(2)

dans laquelle

R a la même signification qu'à la formule (1),

u est un nombre entier compris entre 1 et 20 inclus et

t est un nombre entier compris entre 0 et 20 inclus,

t + u est égal ou supérieur à 3,

formules dans lesquelles le symbole A est un radical de formule :

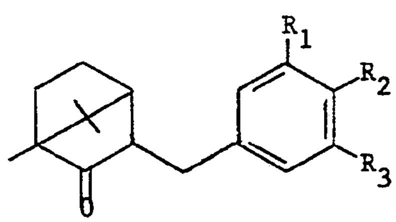

(3)

dans laquelle :

$R_1$ et $R_2$ représentent un atome d'hydrogène, un radical alkyle en $C_1$-$C_6$, un radical alcoxy en $C_1$-$C_6$, hydroxyle, triméthylsilyloxy ou un radical divalent Y de formule :

$$-(O)_n-(CH_2)_p-\underset{\displaystyle R_4}{\underset{|}{CH}}-CH_2- \ ,$$

l'un des deux radicaux $R_1$ et $R_2$ représentant nécessairement le radical divalent Y,

$R_4$ représente un atome d'hydrogène ou un radical alkyle en $C_1$-$C_4$,

n = 0 ou 1,

p est un nombre entier compris entre 1 et 10 inclus,

gel oléoalcoolique, alcoolique ou hydroalcoolique, bâtonnet solide, spray ou aérosol.

13. Composition cosmétique selon l'une quelconque des revendications 6 à 12, se présentant sous forme de composition anti-solaire, caractérisée par le fait qu'elle contient 0,5 à 15% en poids de diorganopolysiloxane de formule (1) ou (2).

14. Composition cosmétique anti-solaire selon revendication 13, caractérisée par le fait qu'elle contient en outre un agent filtrant les rayons UV-B et/ou UV-A, la quantité totale de filtres étant comprise entre 0,5 et 15% en poids par rapport au poids total de la composition.

15. Composition cosmétique selon l'une quelconque des revendications 6 à 11, destinée à être appliquée sur les cheveux, caractérisée par le fait qu'elle se présente sous forme de shampooing, lotion, gel ou émulsion à rincer, à appliquer avant ou après le shampooing, avant ou après coloration ou décoloration, avant ou après permanente, lotion ou gel coiffants ou traitants, lotion ou gel pour le brushing ou la mise en plis, spray de coiffage, laque pour cheveux, composition de permanente, de décoloration ou de coloration et comprend 0,25 à 5% en poids de diorganopolysiloxane de formule (1) ou (2).

16. Composition cosmétique selon l'une quelconque des revendications 6 à 11 se présentant sous forme d'une composition colorée ou non, caractérisée par le fait qu'elle constitue une composition capillaire, un produit de maquillage ou une composition pour les soins ou le traitement de la peau, comprenant 0,25 à 3% en poids de diorganopolysiloxane de formule (1) ou (2).

17. Procédé de protection d'une composition cosmétique contre les rayons ultraviolets, caractérisé par le fait qu'il consiste à incorporer une quantité efficace d'au moins un diorganopolysiloxane à fonction benzilidène-3 camphre de formule (1) ou (2) définie dans l'une quelconque des revendications 1 à 5.

**Patentansprüche**

1. Kosmetische Verwendung von Diorganopolysiloxanen mit einer 3-Benzylidenkampfergruppe, ausgewählt unter jenen der Formel :

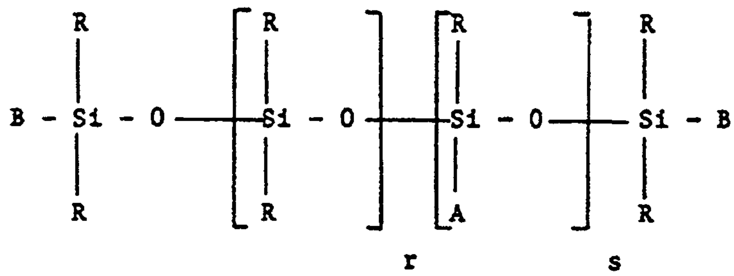

(1)

worin die Reste :

R die gleiche oder verschiedene Bedeutungen haben können und unter den Gruppen $C_1$-$C_{10}$-Alkyl, Phenyl und 3,3,3-Trifluoropropyl ausgewählt sein kinnen, wobei wenigstens 80% der Zahl der Reste R Methylreste sind,

B die gleiche oder verschiedene Bedeutungen haben können und unter den Gruppen R und der Gruppe A gewählt sein können, und wobei

r eine ganze Zahl zwischen einschließlich 0 und 200 sein kann,

s eine ganze Zahl zwischen einschließlich 0 und 50 ist und, wenn s gleich 0 ist, wenigstens einer der zwei Reste B die Gruppe A bedeutet, und jenen der Formel :

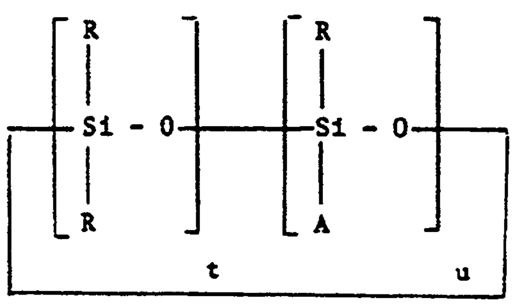

(2)

wobei

R die gleiche Bedeutung wie in der Formel (1) hat

u eine ganze Zahl zwischen einschließlich 1 und 20 ist und

t eine ganze Zahl zwischen einschließlich 0 und 20 ist und

t + u gleich oder größer 3 ist,

wobei in den Formeln der Rest A eine Gruppe der folgenden Formel bedeutet :

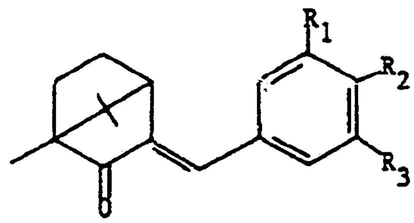

(3)

worin :

$R_1$ und $R_2$ ein Wasserstoffatom, eine $C_1$-$C_6$-Alkylgruppe, eine $C_1$-$C_6$-Alkoxygruppe, eine Hydroxylgruppe, Trimethylsilyloxygruppe oder eine divalente Gruppe Y der Formel :

$$-(O)_n-(CH_2)_p-\underset{\substack{| \\ R_4}}{CH}-CH_2- \quad ,$$

ist, wobei eine der zwei Gruppen $R_1$ und $R_2$ notwendigerweise die divalente Gruppe Y darstellt

$R_4$ ein Wasserstoffatom oder eine $C_1$-$C_4$-Alkylgruppe darstellt,

n gleich 0 oder 1 ist,

p eine ganze Zahl zwischen einschließlich 1 und 10 darstellt, und

$R_3$ ein Wasserstoffatom, eine $C_1$-$C_6$-Alkylgruppe oder eine $C_1$-$C_6$-Alkoxygruppe ist.

2. Kosmetische Verwendung eines statistischen Polymers oder eines Blockpolymers der Formel (1) oder (2) gemäß Anspruch 1 mit wenigstens einem der folgenden Merkmale :

— R ist Methyl,

— B ist Methyl,

— r liegt zwischen einschließlich 5 und 20,

— s liegt zwischen einschließlich 2 und 15,

— t + u liegt zwischen einschließlich 3 und 10,

— $R_1$ ist H oder Methoxy,

— $R_2$ ist H oder Methoxy,

wobei einer der zwei Reste $R_1$ oder $R_2$ die divalente Gruppe Y darstellt, in der n = 0 oder 1, p = 1, $R_4$ = H oder Methyl ist,

— $R_3$ ist H oder Methoxy.

3. Kosmetische Verwendung von Diorganopolysiloxanen mit einer 3-Benzylidenkampfergruppe als Filtermittel für UV-Strahlen mit einer Wellenlänge zwischen 280 und 360 nm, gemäß der Formel :

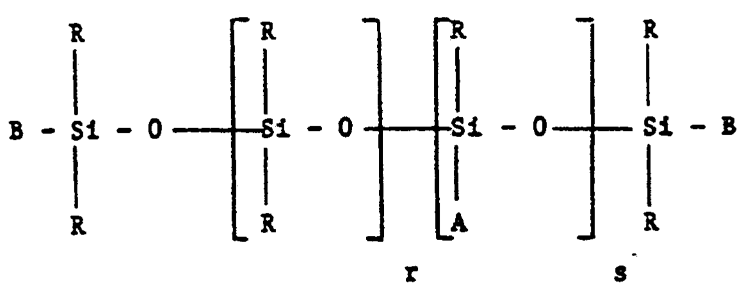

(1)

worin die Reste

R die gleiche oder verschiedene Bedeutungen haben können und unter den Gruppen $C_1$-$C_{10}$-Alkyl, Phenyl und 3,3,3-Trifluoropropyl ausgewählt sein können, wobei wenigstens 80% der Zahl der Reste R Methylreste sind,

B die gleiche oder verschiedene Bedeutungen haben können und unter den Gruppen R und der Gruppe A gewählt sein können,

r eine ganze Zahl zwischen einschließlich 0 und 200 ist,

s eine ganze Zahl zwischen einschließlich 0 und 50 ist und dann, wenn s gleich 0 ist, wenigstens einer der zwei Reste B die Bedeutung A hat, und gemäß der Formel :

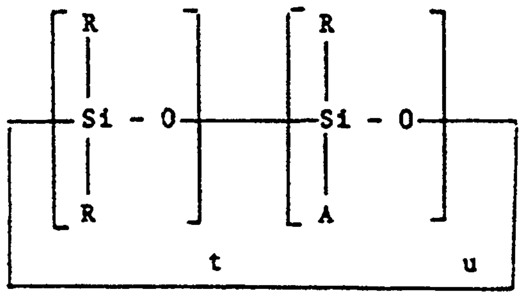

(2)

worin

R die gleiche Bedeutung wie die Formel (1) besitzt,
u eine ganze Zahl zwischen einschließlich 1 und 20 ist
t eine ganze Zahl zwischen einschließlich 0 und 20 ist
t + u gleich oder größer 3 ist,
wobei in der Formel das Symbol A einen Rest der folgenden Formel darstellt:

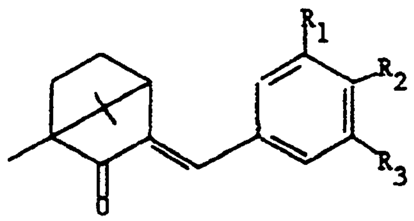

(3)

worin

$R_1$ und $R_2$ ein Wasserstoffatom, einen $C_1$-$C_6$-Alkylrest, einen $C_1$-$C_6$-Alkoxyrest, Hydroxyl, Trimethylsilyloxy oder einen divalenten Rest Y der Formel:

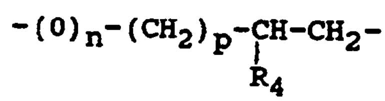

darstellen, wobei einer der zwei Reste $R_1$ und $R_2$ notwendigerweise die divalente Gruppe Y darstellt,
$R_4$ ein Wasserstoffatom oder eine $C_1$-$C_4$-Alkylgruppe darstellt,
n = 0 oder 1
p eine ganze Zahl zwischen einschließlich 1 und 10 ist und
$R_3$ ein Wasserstoffatom, eine $C_1$-$C_6$-Alkylgruppe oder eine $C_1$-$C_6$-Alkoxygruppe darstellt.

4. Kosmetische Verwendung als Filtermittel für UV-Strahlen mit einer Wellenlänge zwischen 280 und 360 nm von Diorganopolysiloxanen der Formeln (1) oder (2) gemäß Anspruch 3 als statistische Polymere oder Blockpolymere, die wenigstens eine der folgenden Merkmale aufweisen:

— R ist Methyl,
— B ist Methyl,
— r liegt zwischen einschließlich 5 und 20,
— s liegt zwischen einschließlich 2 und 15,
— t + u liegt zwischen einschließlich 3 und 10,
— $R_1$ ist H oder Methoxy,
— $R_2$ ist H oder Methoxy,
wobei einer der zwei Reste $R_1$ oder $R_2$ die divalente Gruppe Y bildet, in der n = 0 oder 1, p = 1, $R_4$ = H oder Methyl ist,
— $R_3$ ist H oder Methoxy.

5. Kosmetische Verwendung eines mit 4'-Allyloxy-3-benzylidenkampfers gepfropften Polydimethylsiloxans der Formel (1) gemäß Anspruch 1, in der R und B eine Methylgruppe bezeichnen, r gleich 7 und s gleich 8 sind.

6. Kosmetische Verwendung eines mit 4'-Allyloxy-3'-methoxy-3-benzylidenkampfers gepfropften Polydimethylsiloxans der Formel (1) gemäß Anspruch 1, wobei R und B eine Methylgruppe bezeichnen, r gleich 10 und s gleich 10 sind.

7. Kosmetische Verwendung eines mit 3'-Allyl-4'-methoxy-3-benzylidenkampfers gepfropften Polydimethylsiloxans der Formel (1) gemäß Anspruch 1, worin R und B eine Methylgruppe bezeichnen und r gleich 12 und s gleich 8 sind.

8. Kosmetisches Mittel, dadurch gekennzeichnet, daß es in einem kosmetisch akzeptablen Träger zwischen 0,25 und 15%, bezogen auf das Gesamtgewicht des Mittels, mindestens eines Diorganopolysiloxans mit einer 3-Benzylidenkampfergruppe gemäß der Formel :

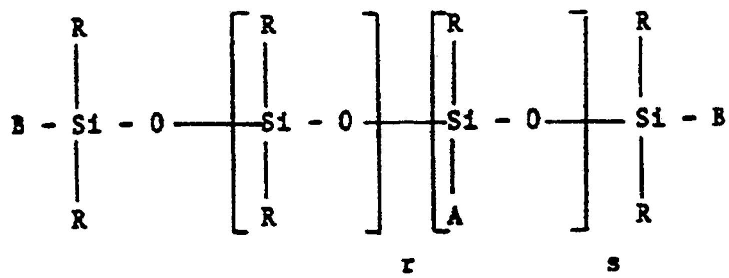

(1)

enthält, worin die Reste

R die gleiche oder verschiedene Bedeutungen haben können und unter $C_1$-$C_{10}$-Alkylgruppe, Phenyl und 3,3,3-Trifluoropropylgruppe gewählt sein können, wobei wenigstens 80% der Zahl der Reste R Methylgruppen sind,

B die gleiche oder verschiedene Bedeutungen haben können und unter den Gruppen R und der Gruppe A gewählt sein können,

r eine ganze Zahl zwischen einschließlich 0 und 200 sein kann,

s eine ganze Zahl zwischen einschließlich 0 und 50 sein kann, wobei dann, wenn s = 0 wenigstens einer der zwei Reste B die Bedeutung A haben kann,

der zwei Reste B die Bedeutung A haben kann, und gemäß der Formel :

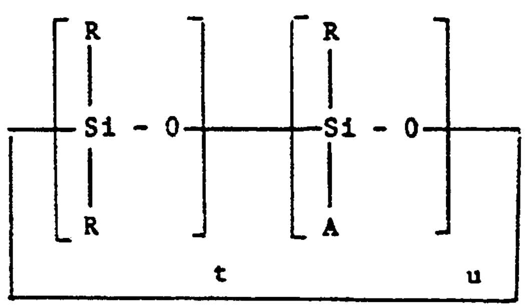

(2)

in der

R die gleiche Bedeutung hat wie in der Formel (1)

u eine ganze Zahl zwischen einschließlich 1 und 20 ist und

t eine ganze Zahl zwischen einschließlich 0 und 20 ist t + u gleich oder größer 3 ist

wobei in den Formeln die Gruppe A der folgenden Formel entspricht :

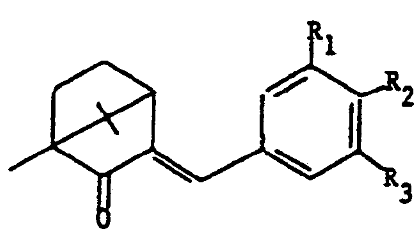

(3)

in der

$R_1$ und $R_2$ ein Wasserstoffatom, eine $C_1$-$C_6$-Alkylgruppe, eine $C_1$-$C_6$-Alkoxygruppe, eine Hydroxylgruppe, Trimethylsilyloxygruppe oder eine divalente Gruppe Y der Formel :

$$-(O)_n-(CH_2)_p-\underset{\displaystyle R_4}{\underset{|}{CH}}-CH_2- \ ,$$

ist, wobei eine der zwei Gruppen $R_1$ und $R_2$ notwendigerweise die divalente Gruppe Y darstellt,
$R_4$ ein Wasserstoffatom oder eine $C_1$-$C_4$-Alkylgruppe darstellt
n gleich 0 oder 1,
p eine ganze Zahl zwischen einschließlich 1 und 10 darstellt,
$R_3$ ein Wasserstoffatom, ein $C_1$-$C_6$-Alkylrest oder ein $C_1$-$C_6$-Alkoxyrest ist.

9. Kosmetisches Mittel nach Anspruch 8, dadurch gekennzeichnet, daß es als statistisches Polymer oder als Blockpolymer ein Diorganopolysiloxan mit einer 3-Benzylidenkampfergruppe der Formel (1) oder (2) enthält, wobei wenigstens eines der folgenden Merkmale gegeben ist : R ist Methyl, B ist Methyl, r liegt zwischen einschließlich 5 und 20, s liegt zwischen einschließlich 2 und 15, t + u liegt zwischen einschließlich 3 und 10, $R_1$ ist H oder Methoxy, $R_2$ ist H oder Methoxy, wobei einer der zwei Reste $R_1$ oder $R_2$ die divalente Gruppe Y darstellt, in der n = 0 oder 1, p = 1, $R_4$ = H oder Methyl ist, und $R_3$ ist H oder Methoxy.

10. Kosmetisches Mittel nach Anspruch 8 oder 9, dadurch gekennzeichnet, daß es ein mit 4'-Allyloxy-3-benzylidenkampfer gepfropftes Polydimethylsiloxan der Formel (1) enthält, in der R und B eine Methylgruppe bezeichnen, r gleich 7 und s gleich 8 sind.

11. Kosmetisches Mittel nach Anspruch 8 oder 9, dadurch gekennzeichnet, daß es ein mit 4'-Allyloxy-3'-methoxy-3-benzylidenkampfer gepfropftes Polydimethylsiloxan der Formel (1) enthält, in der R und B eine Methylgruppe darstellen, r gleich 10 und s gleich 10 sind.

12. Kosmetisches Mittel nach Anspruch 8 oder 9, dadurch gekennzeichnet, daß es ein mit 3'-Allyl-4'-methoxy-3-benzylidenkampfer gepfropftes Polydimethylsiloxan der Formel 1 enthält, in der R und B eine Methylgruppe bezeichnen, r gleich 12 und s gleich 8 sind.

13. Kosmetisches Mittel nach einem der Ansprüche 8 bis 12, dadurch gekennzeichnet, daß es weiters kosmetische Adjuvantien enthält, die unter Verdickungsmitteln, Weichmachern, Feuchtigkeitsmitteln, oberflächenaktiven Mitteln, Konservierungsstoffen, Antischäummitteln, Parfums, Ölen, Wachsen, Lanolin, Monoalkoholen und niederen Polyolen, $C_{12}$-$C_{15}$-Alkoholbenzoaten, Treibmitteln, Farbstoffen und Pigmenten gewählt sind.

14. Kosmetisches Mittel nach einem der Ansprüche 8 bis 13, dadurch gekennzeichnet, daß es in Form öliger, alkoholischer oder oleoalkoholischer Lotionen, als Emulsion, als oleoalkoholisches, alkoholisches oder wasseralkoholisches Gel, als fester Stab, als Spray oder als Aerosol vorliegt.

15. Kosmetisches Mittel nach einem der Ansprüche 8 bis 14, dadurch gekennzeichnet, daß es ein Schutzmittel für die menschliche Haut darstellt und 0,25 bis 3 Gew,-% Diorganopolysiloxan der Formel (1) oder (2) enthält.

16. Kosmetisches Mittel nach einem der Ansprüche 8 bis 14, in Form eines Sonnenschutzmittels, dadurch gekennzeichnet, daß es 0,5 bis 15 Gew.-% Diorganopolysiloxan der Formel (1) oder (2) enthält.

17. Kosmetisches Sonnenschutzmittel nach Anspruch 16, dadurch gekennzeichnet, daß es weiters ein Filtermittel für die Strahlen UV-B und/oder UV-A enthält, wobei die Gesamtmenge der Filterstoffe zwischen 0,5 und 15 Gew.-% bezogen auf das Gesamtgewicht des Mittels liegt.

18. Kosmetisches Mittel nach einem der Ansprüche 8 bis 13, welches dazu bestimmt ist, auf die Haare aufgetragen zu werden, dadurch gekennzeichnet, daß es in Form eines Shampoos, als Lotion, als zu spülendes Gel oder Pulsion vorliegt, die vor oder nach dem Shampoonieren, vor oder nach dem Färben oder Entfärben, vor oder nach einer Dauerwellbehandlung aufgetragen werden, als Lotion oder Gel zum Frisieren oder zur Behandlung, als Lotion oder Gel zum Bürsten oder für die Wasserwelle, als Haarspray, als Haarlack, als Dauerwellmittel, als Entfärbe- oder Färbemittel vorliegt und 0,25 bis 5 Gew.-% Diorganopolysiloxan der Formel (1) oder (2) enthält.

19. Kosmetisches Mittel nach einem der vorhergehenden Ansprüche 8 bis 13, welches in Form eines gefärbten oder nicht gefärbten kosmetischen Mittels vorliegt, dadurch gekennzeichnet, daß es als Haarmittel, Schminkmittel oder als Mittel für die Pflege oder die Behandlung der Haut vorliegt und 0,25 bis 3 Gew.-% Diorganopolysiloxan der Formel (1) oder (2) enthält.

20. Verfahren zur kosmetischen Behandlung der Haut und der natürlichen oder sensibilisierten Haare, um diese gegen Ultraviolettstrahlung zu schützen, dadurch gekennzeichnet, daß es darin besteht, auf die Haut oder auf die Haare eine wirksame Menge eines kosmetischen Mittels aufzutragen, welches wenigstens ein

Diorganopolysiloxan mit einer 3-Benzylidenkampfergruppe der Formel (1) oder (2) gemäß Definition in einem der Ansprüche 3 bis 6 enthält.

21. Verfahren zum Schutz eines kosmetischen Mittels gegen Ultraviolettstrahlen, das einen gegen Licht empfindlichen Bestandteil enthält, dadurch gekennzeichnet, daß es darin besteht, diesem Mittel wenigstens 0,25 bis 3 Gew.-% eines Diorganopolysiloxans mit einer 3-Benzylidenkampfergruppe der Formel (1) oder (2) gemäß einem der Ansprüche 3 bis 6 hinzuzufügen.

**Patentansprüche für folgende Vertragsstaaten : ES**

1. Verfahren zur Herstellung eines kosmetischen Mittels in Form öliger, alkoholischer oder oleoalkoholischer Lotionen als Emulsion, als oleoalkoholisches, alkoholisches oder wasseralkoholisches Gel, als fester Stab, gegebenenfalls als Aerosol konditioniert, zum Schutz der Haut und der Haare gegen Ultraviolettstrahlung,,dadurch gekennzeichnet, daß man in ein kosmetisch akzeptables Milieu zwischen 0,25 und 15%, bezogen auf das Gewicht des Gesamtmittels, wenigstens ein Diorganopolysiloxan mit einer 3-Benzylidenkampfergruppe, ausgewählt unter jenen der Formel :

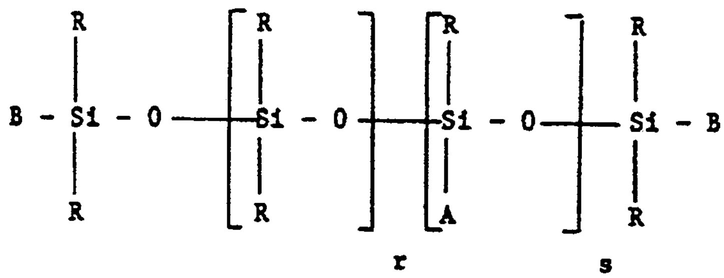

(1)

worin die Reste :

R die gleiche oder verschiedene Bedeutungen haben können und unter den Gruppen $C_1$-$C_{10}$-Alkyl, Phenyl und 3,3,3-Trifluoropropyl ausgewählt sein können, wobei wenigstens 80% der Zahl der Reste R Methylreste sind,

B die gleiche oder verschiedene Bedeutungen haben können und unter den Gruppen R und der Gruppe A gewählt sein kinnen, und wobei

r eine ganze Zahl zwischen einschließlich 0 und 200 sein kann,

s eine ganze Zahl zwischen einschließlich 0 und 50 ist und, wenn s gleich 0 ist, wenigstens einer der zwei Reste B die Gruppe A bedeutet, und der Formel :

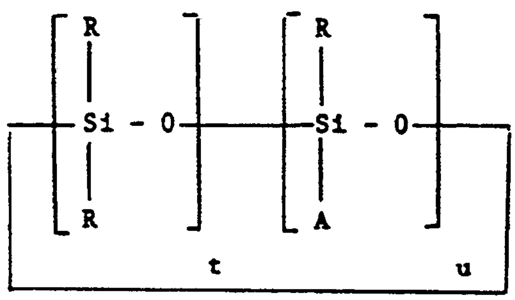

(2)

wobei

R die gleiche Bedeutung wie in der Formel (1) hat

u eine ganze Zahl zwischen einschließlich 1 und 20 ist und

t eine ganze Zahl zwischen einschließlich 0 und 20 ist und

t + u gleich oder größer 3 ist,

wobei in den Formeln der Rest A eine Gruppe der folgenden Formel bedeutet :

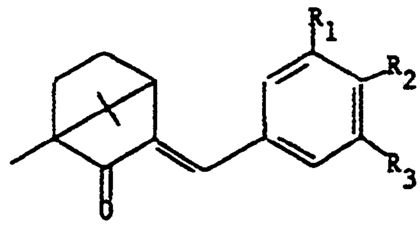

(3)

worin :

$R_1$ und $R_2$ ein Wasserstoffatom, eine $C_1$-$C_6$-Alkylgruppe, eine $C_1$-$C_6$-Alkoxygruppe, eine Hydroxylgruppe, Trimethylsilyloxygruppe oder eine divalente Gruppe Y der Formel :

$$-(O)_n-(CH_2)_p-\underset{\displaystyle R_4}{\underset{|}{CH}}-CH_2- \quad ,$$

ist, wobei eine der zwei Gruppen $R_1$ und $R_2$ notwendigerweise die divalente Gruppe Y darstellt

$R_4$ ein Wasserstoffatom oder eine $C_1$-$C_4$-Alkylgruppe darstellt,

n gleich 0 oder 1 ist,

p eine ganze Zahl zwischen einschließlich 1 und 10 darstellt, und

$R_3$ ein Wasserstoffatom, eine $C_1$-$C_6$-Alkylgruppe oder eine $C_1$-$C_6$-Alkoxygruppe ist, einbringt.

2. Verfahren nach Anspruch 1, dadurch gekennzeichnet, daß ein statistisches Polymer oder ein Blockpolymer der Formel (1) oder (2) mit wenigstens einem der folgenden Merkmale verwendet wird :

— R ist Methyl,
— B ist Methyl,
— r liegt zwischen einschließlich 5 und 20,
— s liegt zwischen einschließlich 2 und 15,
— t + u liegt zwischen einschließlich 3 und 10,
— $R_1$ ist H oder Methoxy,
— $R_2$ ist H oder Methoxy,

wobei einer der zwei Reste $R_1$ oder $R_2$ die divalente Gruppe Y darstellt, in der n = 0 oder 1, p = 1, $R_4$ = H oder Methyl ist,

— $R_3$ ist H oder Methoxy.

3. Verfahren nach Anspruch 1, dadurch gekennzeichnet, daß man ein mit 4'-Allyloxy-3-benzylidenkampfer gepfropftes Polydimethylsiloxan der Formel (1), in der R und B eine Methylgruppe bezeichnen, r gleich 7 und s gleich 8 sind, verwendet.

4. Verfahren nach Anspruch 1, dadurch gekennzeichnet, daß man ein mit 4'-Allyloxy-3'-methoxy-3-benzylidenkampfer gepfropftes Polydimethylsiloxan der Formel (1), wobei R und B eine Methylgruppe bezeichnen, r gleich 10 und s gleich 10 sind, verwendet.

5. Verfahren nach Anspruch 1, dadurch gekennzeichnet, daß man ein mit 3'-Allyl-4'-methoxy-3-benzylidenkampfer gepfropftes Polydimethylsiloxan der Formel (1), worin R und B eine Methylgruppe bezeichnen und r gleich 12 und s gleich 8 sind, verwendet.

6. Kosmetisches Mittel zum Schutz der Haut oder der Haare gegen ultraviolette Strahlung, dadurch gekennzeichnet, daß es in einem kosmetisch akzeptablen Träger zwischen 0,25 und 15%, bezogen auf das Gesamtgewicht des Mittels, mindestens eines Diorganopolysiloxans mit einer 3-Benzylidenkampfergruppe ausgewählt unter jenen der Formel :

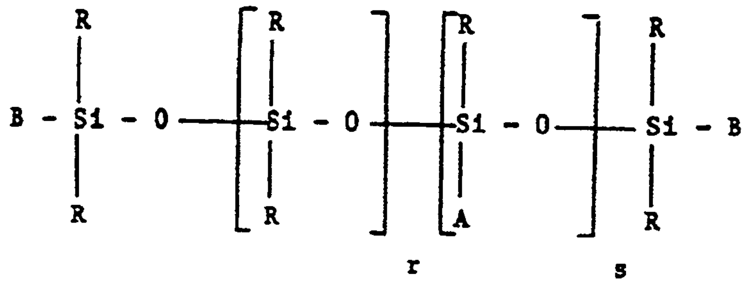

(1)

worin die Reste

R die gleiche oder verschiedene Bedeutungen haben können und unter $C_1$-$C_{10}$-Alkylgruppe, Phenyl und 3,3,3-Trifluoropropylgruppe gewählt sein können, wobei wenigstens 80% der Zahl der Reste R Methylgruppen sind,

B die gleiche oder verschiedene Bedeutungen haben können und unter den Gruppen R und der Gruppe A gewählt sein können,

r eine ganze Zahl zwischen einschließlich 0 und 200 sein kann,

s eine ganze Zahl zwischen einschließlich 0 und 50 sein kann, wobei dann, wenn s = 0 wenigstens einer der zwei Reste B die Bedeutung A haben kann, und jenen der Formel :

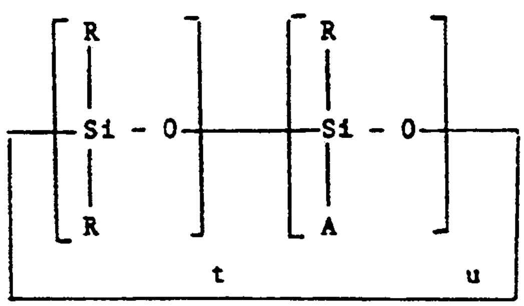

(2)

in der

R die gleiche Bedeutung hat wie in der Formel (1)

u eine ganze Zahl zwischen einschließlich 1 und 20 ist und

t eine ganze Zahl zwischen einschließlich 0 und 20 ist

t + u gleich oder größer 3 ist

wobei in den Formeln die Gruppe A der folgenden Formel entspricht :

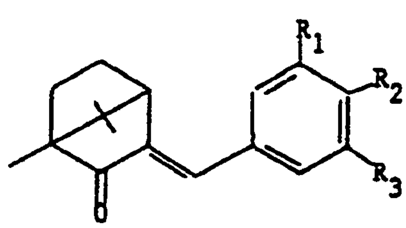

(3)

in der

R1 und R2 ein Wasserstoffatom, eine $C_1$-$C_6$-Alkylgruppe, eine $C_1$-$C_6$-Alkoxygruppe, eine Hydroxylgruppe, Trimethylsilyloxygruppe oder eine divalente Gruppe Y der Formel :

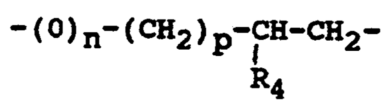 ,

ist, wobei eine der zwei Gruppen $R_1$ und $R_2$ notwendigerweise die divalente Gruppe Y darstellt,

$R_4$ ein Wasserstoffatom oder eine $C_1$-$C_4$-Alkylgruppe darstellt
n gleich 0 oder 1,
p eine ganze Zahl zwischen einschließlich 1 und 10 darstellt,
$R_3$ ein Wasserstoffatom, ein $C_1$-$C_6$-Alkylrest oder ein $C_1$-$C_6$-Alkoxyrest ist, enthält.

7. Kosmetisches Mittel nach Anspruch 6, dadurch gekennzeichnet, daß es als statistisches Polymer oder als Blockpolymer ein Diorganopolysiloxan mit einer 3-Benzylidenkampfergruppe der Formel (1) oder (2) enthält, wobei wenigstens eines der folgenden Merkmale gegeben ist: R ist Methyl, B ist Methyl, r liegt zwischen einschließlich 5 und 20, s liegt zwischen einschließlich 2 und 15, t + u liegt zwischen einschließlich 3 und 10, $R_1$ ist H oder Methoxy, $R_2$ ist H oder Methoxy, wobei einer der zwei Reste $R_1$ oder $R_2$ die divalente Gruppe Y darstellt, in der n = 0 oder 1, p = 1, $R_4$ = H oder Methyl ist, und $R_3$ ist H oder Methoxy.

8. Kosmetisches Mittel nach Anspruch 6 oder 7, dadurch gekennzeichnet, daß es ein mit 4'-Allyloxy-3-benzylidenkampfer gepfropftes Polydimethylsiloxan der Formel (1) enthält, in der R und B eine Methylgruppe bezeichnen, r gleich 7 und s gleich 8 sind.

9. Kosmetisches Mittel nach Anspruch 6 oder 7, dadurch gekennzeichnet, daß es ein mit 4'-Allyloxy-3'-methoxy-3-benzylidenkampfer gepfropftes Polydimethylsiloxan der Formel (1) enthält, in der R und B eine Methylgruppe darstellen, r gleich 10 und s gleich 10 sind.

10. Kosmetisches Mittel nach Anspruch 6 oder 7, dadurch gekennzeichnet, daß es ein mit 3'-Allyl-4'-methoxy-3-benzylidenkampfer gepfropftes Polydimethylsiloxan der Formel 1 enthält, in der R und B eine Methylgruppe bezeichnen, r gleich 12 und s gleich 8 sind.

11. Kosmetisches Mittel nach einem der Ansprüche 6 bis 10, dadurch gekennzeichnet, daß es weiters kosmetische Adjuvantien enthält, die unter Verdickungsmitteln, Weichmachern, Feuchtigkeitsmitteln, oberflächenaktiven Mitteln, Konservierungsstoffen, Antischäummitteln, Parfums, Ölen, Wachsen, Lanolin, Monoalkoholen und niederen Polyolen, $C_{12}$-$C_{15}$-Alkoholbenzoaten, Treibmitteln, Farbstoffen und Pigmenten gewählt sind.

12. Kosmetisches Mittel nach einem der Ansprüche 6 bis 11, dadurch gekennzeichnet, daß es in Form öliger, alkoholischer oder oleoalkoholischer Lotionen, als Emulsion, als oleoalkoholisches, alkoholisches oder wasseralkoholisches Gel, als fester Stab, als Spray oder als Aerosol vorliegt.

13. Kosmetisches Mittel nach einem der Ansprüche 6 bis 12, in Form eines Sonnenschutzmittels, dadurch gekennzeichnet, daß es 0,5 bis 15 Gew.-% Diorganopolysiloxan der Formel (1) oder (2) enthält.

14. Kosmetisches Sonnenschutzmittel nach Anspruch 13, dadurch gekennzeichnet, daß es weiters ein Filtermittel für die Strahlen UV-B und/oder UV-A enthält, wobei die Gesamtmenge der Filterstoffe zwischen 0,5 und 15 Gew.-% bezogen auf das Gesamtgewicht des Mittels liegt.

15. Kosmetisches Mittel nach einem der Anspruche 6 bis 11, welches dazu bestimmt ist, auf die Haare aufgetragen zu werden, dadurch gekennzeichnet, daß es in Form eines Shampoos, als Lotion, als zu spulendes Gel oder Emulsion vorliegt, die vor oder nach dem Shampoonieren, vor oder nach dem Färben oder Entfärben, vor oder nach einer Dauerwellbehandlung aufgetragen werden, als Lotion oder Gel zum Frisieren oder zur Behandlung, als Lotion oder Gel zum Bürsten oder für die Wasserwelle, als Haarspray, als Haarlack, als Dauerwellmittel, als Entfärbe- oder Färbemittel vorliegt und 0,25 bis 5 Gew.-% Diorganopolysiloxan der Formel (1) oder (2) enthält.

16. Kosmetisches Mittel nach einem der vorhergehenden Ansprüche 6 bis 11, welches in Form eines gefärbten oder nicht gefärbten kosmetischen Mittels vorliegt, dadurch gekennzeichnet, daß es als Haarmittel, Schminkmittel oder als Mittel für die Pflege oder die Behandlung der Haut vorliegt und 0,25 bis 3 Gew.-% Diorganopolysiloxan der Formel (1) oder (2) enthält.

17. Verfahren zum Schutz eines kosmetischen Mittels gegen Ultraviolettstrahlen, dadurch gekennzeichnet, daß es darin besteht, diesem Mittel eine wirksame Menge wenigstens eines Diorganopolysiloxans mit einer 3-Benzylidenkampfergruppe der Formel (1) oder (2) gemäß einem der Ansprüche 1 bis 5 zuzufügen.

## Claims

1. Use in cosmetics of diorganopolysiloxanes containing a 3-benzylidenecamphor group, chosen from those of formula :

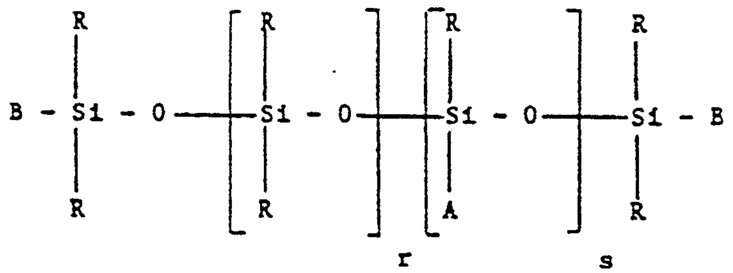 (1)

in which the symbols :

R, which may be identical or different, are chosen from $C_1$-$C_{10}$-alkyl, phenyl and 3,3,3-trifluoropropyl radicals, at least 80% by number of the radicals R being methyl radicals,

B, which may be identical or different, are chosen from the radicals R and the radical A,

r is an integer chosen between 0 and 200 inclusive,

s is an integer chosen between 0 and 50 inclusive and if s is 0 at least one of the two symbols B denotes A, and those of formula :

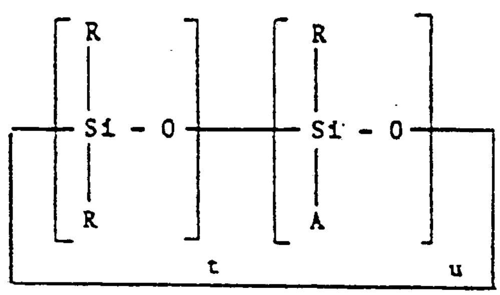 (2)

in which

R has the same meaning as in formula (1),

u is an integer between 1 and 20 inclusive and

t is an integer between 0 and 20 inclusive, and

t + u is equal to or greater than 3,

in which formulae the symbol A is a radical of formula :

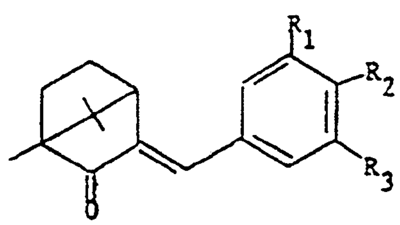 (3)

in which :

$R_1$ and $R_2$ represent a hydrogen atom, a $C_1$-$C_6$-alkyl radical, a $C_1$-$C_6$-alkoxy, hydroxyl or trimethylsilyloxy radical or a divalent radical Y of formula :

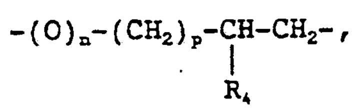

one of the two radicals $R_1$ and $R_2$ necessarily representing the divalent radical Y,

$R_4$ represents a hydrogen atom or a $C_1$-$C_4$-alkyl radical,

n is 0 or 1,

p is an integer between 1 and 10 inclusive, and

$R_3$ represents a hydrogen atom or a $C_1$-$C_6$-alkyl or $C_1$-$C_6$-alkoxy radical.

2. Use in cosmetics of a random or block polymer of formula (1) or (2) according to Claim 1, having at least one of the following characteristics :

— R is methyl,
— B is methyl,
— r is between 5 and 20 inclusive,
— s is between 2 and 15 inclusive,
— t + u is between 3 and 10 inclusive,
— $R_1$ is H or methoxy,
— $R_2$ is H or methoxy, one of the two radicals $R_1$ or $R_2$ representing the divalent radical Y in which n = 0 or 1, p = 1 and R, = H or methyl, and
— $R_3$ is H or methoxy.

3. Use in cosmetics, as agents filtering W radiation of wavelengths between 280 and 360 nm, of diorganopolysiloxanes containing a 3-benzylidenecamphor group, chosen from those of formula :

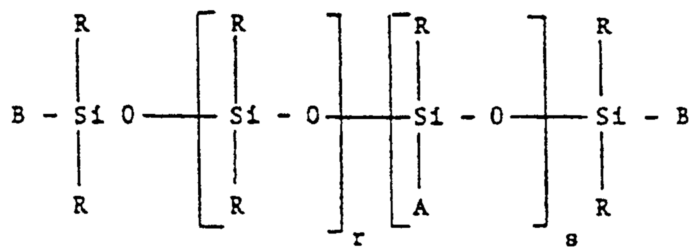
(1)

in which the symbols :

R, which may be identical or different, are chosen from $C_1$-$C_{10}$-alkyl, phenyl and 3,3,3-trifluoropropyl radicals, at least 80% by number of the radicals R being methyl radicals,
B, which may be identical or different, are chosen from the radicals R and the radical A,
r is an integer chosen between 0 and 200 inclusive,
s is an integer chosen between 0 and 50 inclusive and if s is 0 at least one of the two symbols B denotes A, and those of formula :

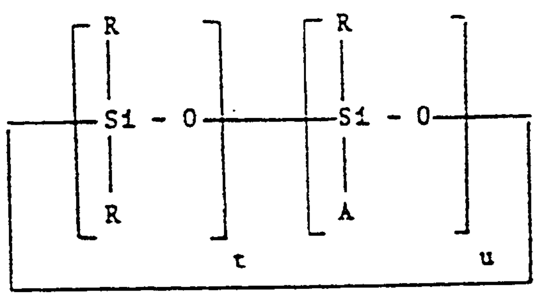
(2)

in which

R has the same meaning as in formula (1),
u is an integer between 1 and 20 inclusive and
t is an integer between 0 and 20 inclusive, and
t + u is equal to or greater than 3, in which formulae the symbol A is a radical of formula :

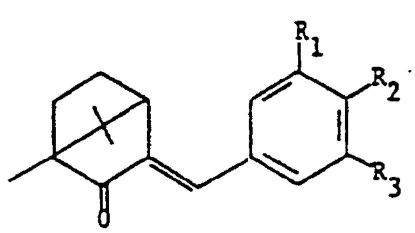
(3)

in which :

$R_1$ and $R_2$ represent a hydrogen atom, a $C_1$-$C_6$-alkyl radical, a $C_1$-$C_6$-alkoxy, hydroxyl or trimethylsilyloxy radical or a divalent radical Y of formula :

$$-(O)_n-(CH_2)_p-\underset{\displaystyle R_4}{\underset{|}{CH}}-CH_2-,$$

one of the two radicals $R_1$ and $R_2$ necessarily representing the divalent radical Y,
$R_4$ represents a hydrogen atom or a $C_1$-$C_4$-alkyl radical,
n is 0 or 1,
p is an integer between 1 and 10 inclusive, and
$R_3$ represents a hydrogen atom or a $C_1$-$C_6$-alkyl or $C_1$-$C_6$-alkoxy radical.

4. Use in cosmetics, as agents filtering UV radiation of wavelengths between 280 and 360 nm, of random or block diorganopolysiloxanes of formulae (1) or (2) according to Claim 3, having at least one of the following characteristics :

— R is methyl,
— B is methyl,
— r is between 5 and 20 inclusive,
— s is between 2 and 15 inclusive,
— t + u is between 3 and 10 inclusive,
— $R_1$ is H or methoxy,
— $R_2$ is H or methoxy,

one of the two radicals $R_1$ or $R_2$ representing the divalent radical Y in which n = 0 or 1, p = 1 and $R_4$ = H or methyl, and

— $R_3$ is H or methoxy.

5. Use in cosmetics of a polydimethylsiloxane containing 4′-allyloxy-3-benzylidenecamphor grafts of formula (1) according to Claim 1, in which R and B denote methyl, r is 7 and s is 8.

6. Use in cosmetics of a polydimethylsiloxane containing 4′-allyloxy-3′-methoxy-3-benzylidenecamphor grafts of formula (1) according to Claim 1, in which R and B denote methyl, r is 10 and s is 10.

7. Use in cosmetics of a polydimethylsiloxane containing 3′-allyl-4′-methoxy-3-benzylidenecamphor grafts of formula (1) according to Claim 1, in which R and B denote methyl, r is 12 and s is 8.

8. Cosmetic composition, characterised in that it comprises, in a cosmetically acceptable carrier, between 0.25 and 15%, relative to the total weight of the composition, of at least one diorganopolysiloxane containing a 3-benzylidenecamphor group, chosen from those of formula :

$$B-\underset{R}{\overset{R}{Si}}-O-\left[\underset{R}{\overset{R}{Si}}-O\right]_r-\left[\underset{A}{\overset{R}{Si}}-O\right]_s-\underset{R}{\overset{R}{Si}}-B \quad (1)$$

in which the symbols :

R, which may be identical or different, are chosen from $C_1$-$C_{10}$-alkyl, phenyl and 3,3,3-trifluoropropyl radicals, at least 80% by number of the radicals R being methyl radicals,
B, which may be identical or different, are chosen from the radicals R and the radical A,
r is an integer chosen between 0 and 200 inclusive,
s is an integer chosen between 0 and 50 inclusive and if s is 0 at least one of the two symbols B denotes A, and those of formula :

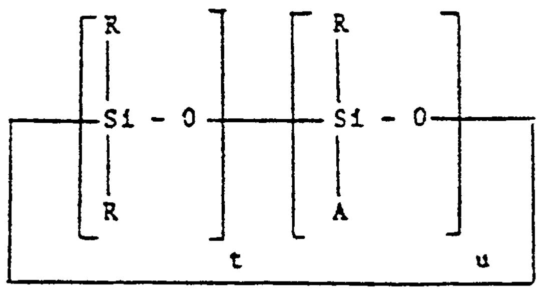

(2)

in which

R has the same meaning as in formula (1),

u is an integer between 1 and 20 inclusive and

t is an integer between 0 and 20 inclusive, and

t + u is equal to or greater than 3,

in which formulae the symbol A is a radical of formula :

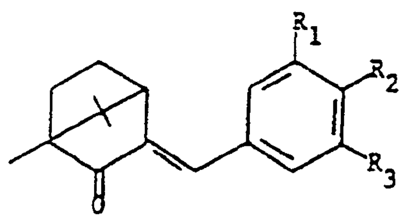

(3)

in which :

$R_1$ and $R_2$ represent a hydrogen atom, a $C_1$-$C_6$-alkyl radical, a $C_1$-$C_6$-alkoxy, hydroxyl or trimethylsilyloxy radical or a divalent radical Y of formula :

$$-(O)_n-(CH_2)_p-\underset{\displaystyle R_4}{\underset{|}{CH}}-CH_2-,$$

one of the two radicals $R_1$ and $R_2$ necessarily representing the divalent radical Y,

$R_4$ represents a hydrogen atom or a $C_1$-$C_4$-alkyl radical,

n is 0 or 1,

p is an integer between 1 and 10 inclusive, and

$R_3$ represents a hydrogen atom or a $C_1$-$C_6$-alkyl or $C_1$-$C_6$-alkoxy radical.

9. Cosmetic composition according to Claim 8, characterised in that it comprises a random or block diorganopolys iloxane containing a 3-benzylidenecamphor group of formula (1) or (2), having at least one of the following characteristics :

R is methyl, B is methyl, r is between 5 and 20 inclusive, s is between 2 and 15 inclusive, t + u is between 3 and 10 inclusive, $R_1$ is H or methoxy, $R_2$ is H or methoxy, one of the two radicals $R_1$ or $R_2$ representing a divalent radical Y in which n = 0 or 1, p = 1 and $R_4$ = H or methyl, and $R_3$ is H or methoxy.

10. Cosmetic composition according to Claim 8 or 9, characterised in that it comprises a polydimethylsiloxane containing 4′-allyloxy-3′-benzylidenecamphor grafts of formula (1), in which R and B denote a methyl group, r is 7 and s is 8.

11. Cosmetic composition according to Claim 8 or 9, characterised in that it comprises a polydimethylsiloxane containing 4′-allyloxy-3′-methoxy-3-benzylidenecamphor grafts of formula (1), in which R and B denote a methyl group, r is 10 and s is 10.

12. Cosmetic composition according to Claim 8 or 9, characterised in that it comprises a polydimethylsiloxane containing 3′-allyl-4′-methoxy-3-benzylidenecamphor grafts of formula (1), in which R and B denote a methyl group, r is 12 and s is 8.

13. Cosmetic composition according to any one of Claims 8 to 12, characterised in that it also contains cosmetic adjuvants chosen from thickeners, softeners, humectants, surfactants, preservatives, anti-foams, perfumes, oils, waxes, lanolin, lower monoalcohols and polyols, $C_{12}$-$C_{15}$-alcohol benzoates, propellants, dyes and pigments.

14. Cosmetic composition according to any one of Claims 8 to 13, characterised in that it is in the form of an oily, alcoholic or oily alcoholic lotion, an oily alcoholic, alcoholic or aqueous alcoholic emulsion or gel, a solid stick, a spray or an aerosol.

15. Cosmetic composition according to any one of Claims 8 to 14, characterised in that it is a composition which protects the human epidermis and contains 0.25 to 3% by weight of diorganopolysiloxane of formula (1) or (2).

16. Cosmetic composition according to any one of Claims 8 to 14, which is in the form of a sun-screen composition, characterised in that it contains 0.5 to 15% by weight of diorganopolysiloxane of formula (1) or (2).

17. Sun-screen cosmetic composition according to Claim 16, characterised in that it also contains an agent filtering UV-B and/or UV-A radiation, the total amount of filters being between 0.5 and 15% by weight relative to the total weight of the composition.

18. Cosmetic composition according to any one of Claims 8 to 13, intended for application to the hair, characterised in that it is in the form of a shampoo, lotion, gel or emulsion to be rinsed off, to be applied before or after shampooing, before or after dyeing or bleaching and before or after permanent waving, any hair dressing or treatment lotion or gel, a lotion or gel for blow-drying or hair setting, a hair spray, hair lacquer or composition for permanent waving, bleaching or dyeing and comprises 0.25 to 5% by weight of diorganopolysiloxane of formula (1) or (2).

19. Cosmetic composition according to any one of Claims 8 to 13, which is in the form of a coloured or colourless cosmetic composition, characterised in that it is a composition for the hair, a make-up product or a composition for the care or treatment of the skin, comprising 0.25 to 3% by weight of diorganopolysiloxane of formula (1) or (2).

20. Method for cosmetic treatment of the skin and natural or sensitised hair with a view to protecting them against ultraviolet radiation, characterised in that it consists in applying to the skin or the hair an effective amount of a cosmetic composition containing at least one diorganopolysiloxane containing a 3-benzylidenecamphor group of formula (1) or (2) defined in any one of Claims 3 to 6.

21. Method for the protection of a cosmetic composition containing a light-sensitive constituent against ultraviolet radiation, characterised in that it consists in incorporating in this composition at least 0.25 to 3% by weight of a diorganopolysiloxane containing a 3-benzylidenecamphor group of formula (1) or (2) defined in any one of Claims 3 to 6.

**Claims for the following Contracting State : ES**

1. process for the preparation of a cosmetic composition in the form of an oily, alcoholic or oily alcoholic lotion, oily alcoholic, alcoholic or aqueous alcoholic emulsion or gel, or a solid stick, which is optionally packaged as an aerosol, for the protection of the skin and hair against ultraviolet radiation, characterised in that at least one diorganopolysiloxane containing a 3-benzylidenecamphor group, chosen from those of formula :

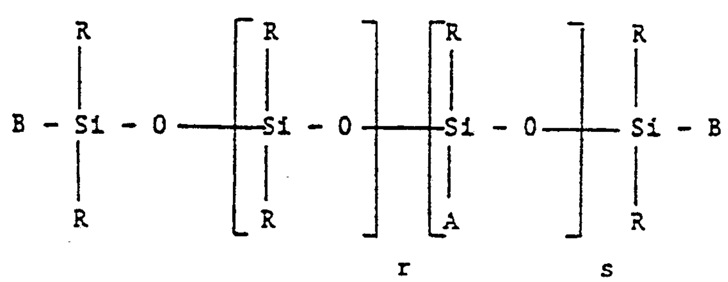

(1)

in which the symbols :

R, which may be identical or different, are chosen from $C_1$-$C_{10}$-alkyl, phenyl and 3,3,3-trifluoropropyl radicals, at least 80% by number of the radicals R being methyl radicals,

B, which may be identical or different, are chosen from the radicals R and the radical A,

r is an integer chosen between 0 and 200 inclusive,

s is an integer chosen between 0 and 50 inclusive and if s is 0 at least one of the two symbols B denotes A, and those of formula :

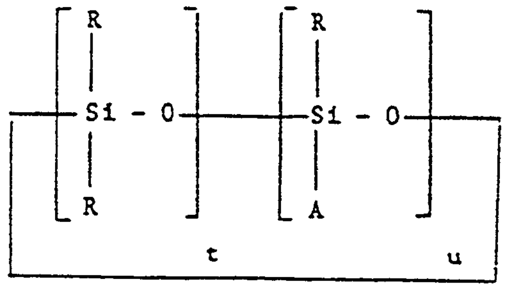

(2)

in which

R has the same meaning as in formula (1),
u is an integer between 1 and 20 inclusive and
t is an integer between 0 and 20 inclusive, and
t + u is equal to or greater than 3,
in which formulae the symbol A is a radical of formula :

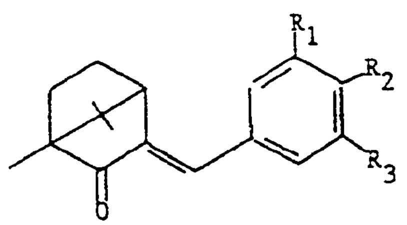

(3)

in which :

$R_1$ and $R_2$ represent a hydrogen atom, a $C_1$-$C_6$-alkyl radical, a $C_1$-$C_6$-alkoxy, hydroxyl or trimethylsilyloxy radical or a divalent radical Y of formula :

$$-(O)_n-(CH_2)_p-\underset{\displaystyle R_4}{\underset{|}{CH}}-CH_2-,$$

one of the two radicals $R_1$ and $R_2$ necessarily representing the divalent radical Y,
$R_4$ represents a hydrogen atom or a $C_1$-$C_4$-alkyl radical,
n is 0 or 1,
p is an integer between 1 and 10 inclusive, and
$R_3$ represents a hydrogen atom or a $C_1$-$C_6$-alkyl or $C_1$-$C_6$-alkoxy radical,
is introduced into a cosmetically acceptable medium, in proportions of between 0.25 and 15% relative to the total weight of the composition.

2. Process according to Claim 1, characterised in that a random or block polymer of formula (1) or (2) is used which has at least one of the following characteristics :

— R is methyl,
— B is methyl,
— r is between 5 and 20 inclusive,
— s is between 2 and 15 inclusive,
— t + u is between 3 and 10 inclusive,
— $R_1$ is H or methoxy,
— $R_2$ is H or methoxy,
one of the two radicals $R_1$ or $R_2$ representing the divalent radical Y in which n = 0 or 1, p = 1 and R, = H or methyl, and
— $R_3$ is H or methoxy.

3. Process according to Claim 1, characterised in that a polydimethylsiloxane containing 4'-allyloxy-3-benzylidenecamphor grafts of formula (1) is used in which R and B denote methyl, r is 7 and s is 8.

4. Process according to Claim 1, characterised in that a polydimethylsiloxane containing 4'-allyloxy-3'-methoxy-3-benzylidenecamphor grafts of formula (1) is used in which R and B denote methyl, r is 10 and s is 10.

5. Process according to Claim 1, characterised in that a polydimethylsiloxane containing 3'-allyl-4'methoxy-3-benzylidenecamphor grafts of formula (1) is used in which R and B denote methyl, r is 12 and s is 8.

6. Cosmetic composition intended for the protection of the skin or hair against ultraviolet radiation, characterised in that it comprises, in a cosmetically acceptable carrier, at least in an amount of between 0.25 and 15% by weight relative to the total weight of the composition, a diorganopolysiloxane containing a 3-benzylidenecamphor group chosen from those of formula :

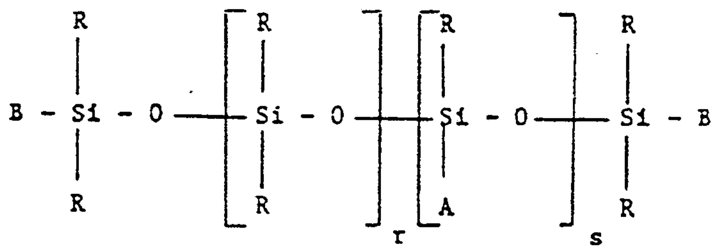

(1)

in which the symbols :

R, which may be identical or different, are chosen from $C_1$-$C_{10}$-alkyl, phenyl and 3,3,3-trifluoropropyl radicals, at least 80% by number of the radicals R being methyl radicals,

B, which may be identical or different, are chosen from the radicals R and the radical A,

r is an integer chosen between 0 and 200 inclusive,

s is an integer chosen between 0 and 50 inclusive and if s is 0 at least one of the two symbols B denotes A, and those of formula :

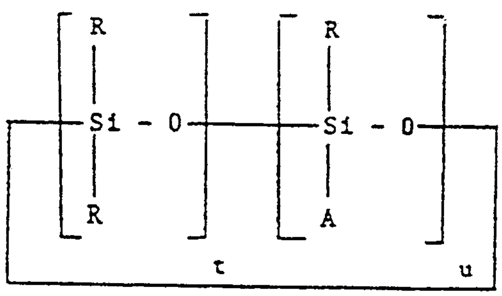

(2)

in which

R has the same meaning as in formula (1),

u is an integer between 1 and 20 inclusive and

t is an integer between 0 and 20 inclusive, and

t + u is equal to or greater than 3,

in which formulae the symbol A is a radical of formula :

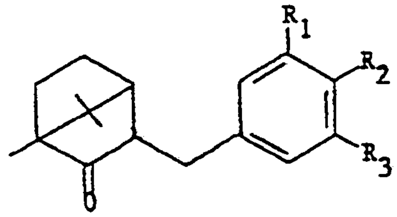

(3)

in which :

$R_1$ and $R_2$ represent a hydrogen atom, a $C_1$-$C_6$-alkyl radical, a $C_1$-$C_6$-alkoxy, hydroxyl or trimethyls ilyloxy radical or a divalent radical Y of formula :

$$-(O)_n-(CH_2)_p-\underset{\displaystyle R_4}{\underset{|}{CH}}-CH_2-,$$

one of the two radicals $R_1$ and $R_2$ necessarily representing the divalent radical Y,
$R_4$ represents a hydrogen atom or a $C_1$-$C_4$-alkyl radical,
n is 0 or 1,
p is an integer between 1 and 10 inclusive, and
$R_3$ represents a hydrogen atom or a $C_1$-$C_6$-alkyl or $C_1$-$C_6$-alkoxy radical.

7. Cosmetic composition according to Claim 6, characterised in that it comprises a random or block diorganopolysiloxane containing a 3-benzylidenecamphor group of formula (1) or (2), having at least one of the following characteristics :

R is methyl, B is methyl, r is between 5 and 20 inclusive, s is between 2 and 15 inclusive, t + u is between 3 and 10 inclusive, $R_1$ is H or methoxy, $R_2$ is H or methoxy, one of the two radicals $R_1$ or $R_2$ representing a divalent radical Y in which n = 0 or 1, p = 1 and R, = H or methyl, and R, is H or methoxy.

8. Cosmetic composition according to Claim 6 or 7, characterised in that it comprises a polydimethylsiloxane containing 4′-allyloxy-3-benzylidenecamphor grafts of formula (1), in which R and B denote a methyl group, r is 7 and s is 8.

9. Cosmetic composition according to Claim 6 or 7, characterised in that it comprises a polydimethylsiloxane containing 4′-allyloxy-3′-methoxy-3-benzylidenecamphor grafts of formula (1), in which R and B denote a methyl group, r is 10 and s is 10.

10. Cosmetic composition according to Claim 6 or 7, characterised in that it comprises a polydimethylsiloxane containing 3′-allyl-4′-methoxy-3-benzylidenecamphor grafts of formula (1), in which R and B denote a methyl group, r is 12 and s is 8.

11. Cosmetic composition according to any one of Claims 6 to 10, characterised in that it also contains cosmetic adjuvants chosen from thickeners, softeners, humectants, surfactants, preservatives, anti-foams, perfumes, oils, waxes, lanolin, lower monoalcohols and polyols, $C_{12}$-$C_{15}$-alcohol benzoates, propellants, dyes and pigments.

12. Cosmetic composition according to any one of Claims 6 to 11, characterised in that it is in the form of an oily, alcoholic or oily alcoholic lotion, an oily alcoholic, alcoholic or aqueous alcoholic emulsion or gel, a solid stick, a spray or an aerosol.

13. Cosmetic composition according to any one of Claims 6 to 12, which is in the form of a sun-screen composition, characterised in that it contains 0.5 to 15% by weight of diorganopolysiloxane of formula (1) or (2).

14. Sun-screen cosmetic composition according to Claim 13, characterised in that it also contains an agent filtering UV-B and/or UV-A radiation, the total amount of filters being between 0.5 and 15% by weight relative to the total weight of the composition.

15. Cosmetic composition according to any one of Claims 6 to 11, intended for application to the hair, characterised in that it is in the form of a shampoo, lotion, gel or emulsion to be rinsed off, to be applied before or after shampooing, before or after dyeing or bleaching and before or after permanent waving, any hair dressing or treatment lotion or gel, a lotion or gel for blow-drying or hair setting, a hair spray, hair lacquer or composition for permanent waving, bleaching or dyeing and comprises 0.25 to 5% by weight of diorganopolysiloxane of formula (1) or (2).

16. Cosmetic composition according to any one of Claims 6 to 11, which is in the form of a coloured or colourless cosmetic composition, characterised in that it is a composition for the hair, a make-up product or a composition for the care or treatment of the skin, comprising 0.25 to 3% by weight of diorganopolysiloxane of formula (1) or (2).

17. Method for the protection of a cosmetic composition against ultraviolet radiation, characterised in that it consists in incorporating an effective amount of at least one diorganopolysiloxane containing a 3-benzylidenecamphor group of formula (1) or (2) defined in any one of Claims 1 to 5.